Europäisches Patentamt

(19) European Patent Office
Office européen des brevets

(11) Veröffentlichungsnummer: **0 104 466**
**A1**

(12) **EUROPÄISCHE PATENTANMELDUNG**

(21) Anmeldenummer: 83108494.2

(22) Anmeldetag: 29.08.83

(51) Int. Cl.³: **A 61 N 5/06**

(30) Priorität: 01.09.82 DE 3232537
09.12.82 DE 3245654
09.12.82 DE 3245655
18.06.83 DE 8317832 U

(43) Veröffentlichungstag der Anmeldung:
04.04.84 Patentblatt 84/14

(84) Benannte Vertragsstaaten:
AT BE CH DE FR GB IT LI LU NL SE

(71) Anmelder: Kerschgens, Johann Josef
Prinz-Ludwig-Strasse 5
D-8918 Diessen am Ammersee(DE)

(72) Erfinder: Kerschgens, Johann Josef
Prinz-Ludwig-Strasse 5
D-8918 Diessen am Ammersee(DE)

(74) Vertreter: Patentanwälte Dr. Solf & Zapf
c/o Patentanwälte Dr. Hasse Dr. Solf & Zapf Asamstrasse
8
D-8000 München 90(DE)

(54) Bestrahlungsgerät.

(57) Bestrahlungsgerät, kombiniert aus einem Gebläse (140) und einer Strahlungsquelle (38), die ultraviolette Strahlung (UV-Strahlung) abgibt und über einen Vorwiderstand an die Netzspannung eine Wechselspannungsquelle angeschlossen ist, wobei die Strahlungsquelle mit dem vom Gebläse, insbesondere eines Haarföns (141) erzeugten Luftstroms zusammenwirkt. Der Vorwiderstand ist im wesentlichen kapazitiv, und die Strahlungsquelle (33) ist im Luftstrom des Gebläses angeordnet, wobei der Luftstrom die Strahlungsquelle derart kühlt, daß der Momentanwert ihrer Wiederzündspannung ≤ dem Momentanwert der Netzspannung ist.

FIG.10

EP 0 104 466 A1

0104466


M 149/+I/rk


Johann Josef Kerschgens

Prinz-Ludwig-Straße 5, 8918 Diessen am Ammersee


Bestrahlungsgerät


Die Erfindung betrifft ein Bestrahlungsgerät, kombiniert aus einem Gebläse und einer Strahlungsquelle, die ultraviolette Strahlung (UV-Strahlung) abgibt und über einen Vorwiderstand an die Netzspannung eine Wechselspannungsquelle angeschlossen ist, wobei die Strahlungsquelle mit dem vom Gebläse erzeugten Luftstrom zusammenwirkt.

Bestrahlungsgeräte mit ultraviolette Strahlung (UV-Strahlung) abgebenden Quecksilber-Dampflampen sowie durch elektrischen Stromfluß aufheizbare Stäbe zur Abgabe von infraroter Strahlung (IR-Strahlung) sind bekannt. Die ultravioletten Strahlen der Quecksilber-Dampflampe werden zur Hautbestrahlung eingesetzt, um therapeutische und kosmetische Wirkung zu erzielen, und/oder um die Widerstandskraft des Körpers zu erhöhen. Ultraviolette Strahlung wird nach ihrer Wellenlänge in UV-A-, UV-B- und UV-C-Strahlung unterteilt. UV-A-Strahlen haben eine Wellenlänge von 315 - 400 nm. UV-B-Strahlen weisen eine Wellenlänge

- 2 -

von 280 - 315 nm auf und UV-C-Strahlen liegen im Wellenbereich zwischen 200 - 280 nm. Die Wirkung der UV-Strahlung ist vielfältig. Die menschliche Haut beispielsweise reagiert auf UV-B- und UV-A-Strahlen durch Bräunung. Mit einer UV-C-Strahlung kann Ozon erzeugt werden; darüber hinaus zerstört sie außerdem. Mikroorganismen, wie Bakterien, Viren, Sporen, Hefen, Algen, Protozoen und Schimmelpilze. Die ultravioletten Strahlen lösen in der menschlichen Haut photobiologische Effekte aus. Dabei entsteht durch kurzwellige UV-Strahlung das sogenannte UV-Erythen, als dessen Folge nach einigen Tagen eine Bräunung der Haut (indirekte Pigmentierung) auftritt. Dagegen führen hohe Dosen im langwelligeren UV-Bereich zu einer direkten Pigmentierung, die ohne Erythenbildung erreicht wird. Das Maximum der Hautempfindlichkeit für die direkte Pigmentierung liegt bei einer Wellenlänge von 360 nm.

Neben der Wirkung der UV-Strahlung zur Bräunung der Haut hat sich gezeigt, daß ein auf eine Hauterkrankung (z. B. Schuppenflechte) geleiteter Luftstrom in Verbindung mit einer UV-Bestrahlung zu einer außerordentlich guten Heilwirkung und schnellen Ausmerzung der Erkrankung führt.

Es ist ein Bestrahlungsgerät bekannt, bei dem ein Fön in Verbindung mit einer UV-Lampe verwendet wird. Da die UV-Lampe dieses bekannten Gerätes über einen Ohm'schen Vorwiderstand betrieben wird, erfolgt nach längerer Betriebszeit durch Wärmeleitung und

- 3 -

insbesondere durch Wärmestrahlung ein Aufheizen der UV-Lampe. Es hat sich hierbei herausgestellt, daß die Wirksamkeit des Gerätes bei längerer Betriebsdauer der UV-Lampe nachläßt, was zu langen Behandlungszeiten führt und darüber hinaus eine reproduzierbare Behandlungstherapie erschwert. Weiterhin ist von Nachteil, daß die von dem Ohm'schen Vorwiderstand abgestrahlte Wärme zu einer unangenehmen Erhitzung der Behandlungsstelle führt, wodurch der Benutzer geneigt ist, das Bestrahlungsgerät relativ weit entfernt von der Behandlungsstelle zu plazieren, was aber die Wirksamkeit der Behandlung herabsetzt.

Es ist weiterhin ein Bestrahlungsgerät bekannt, das eine UV-Lampe in Kombination mit einem oder mehreren IR-Strahler(n) aufweist. Als UV-Lampe wird meist eine Quecksilberdampflampe und als IR-Strahler ein Widerstandsheizstab verwendet. Dieser Heizstab dient als Vorwiderstand für die UV-Lampe. Hierdurch wird der UV-Lampenstrom begrenzt und der Arbeitspunkt für die UV-Lampe festgelegt. Während des Betriebes der UV-Lampe heizt sich auch der IR-Strahler (Heizstab) auf, da diese beiden Bauteile in Reihe geschaltet sind. Somit treten auch hier die gleichen oben genannten Nachteile auf, und darüber hinaus gibt dieses bekannte Gerät keinen für den Behandlungserfolg notwendigen Luftstrom ab.

Aufgabe der Erfindung ist es demgegenüber, ein Bestrahlungsgerät der eingangs genannten Art derart zu verbessern, daß es sich durch eine kompakte, leichte

- 4 -

und kostengünstige Bauweise auszeichnet, aus wenigen Bauteilen besteht, für einen Dauerbetrieb geeignet ist und über die gesamte Brenndauer der UV-Lampe eine gleichmäßige Behandlungswirkung ermöglicht sowie einen relativ kleinen Bestrahlungsabstand zuläßt.

Diese Aufgabe wird erfindungsgemäß dadurch gelöst, daß der Vorwiderstand im wesentlichen kapazitiv ist, und daß die Strahlungsquelle im Luftstrom des Gebläses angeordnet ist, wobei der Luftstrom die Strahlungsquelle derart kühlt, daß der Momentanwert ihrer Wiederzündspannung $\leq$ dem Momentanwert der Netzspannung ist.

Vorteilhafte Weiterbildungen der Erfindung sind durch die Unteransprüche gekennzeichnet.

Die Erfindung basiert auf der Erkenntnis, daß sich der Arbeitspunkt der UV-Lampe durch ihre Erhitzung verschiebt, wodurch sich die Wellenlänge und die Leistung der abgegebenen UV-Strahlung verändert. Zur Erzielung guter Behandlungserfolge ist es jedoch notwendig, daß die UV-Strahlung möglichst nur aus einem relativ schmalen, bestimmten Frequenzspektrum der UV-Strahlung zusammengesetzt ist. Mit dem erfindungsgemäßen Bestrahlungsgerät ist die Abgabe dieses gewünschten Strahlenspektrums möglich, da der Vorwiderstand der UV-Lampe kapazitiv ausgebildet ist und sich somit während des Betriebes nur unwesentlich erwärmt. Dadurch erfolgt jedoch keine Aufheizung - und damit Arbeitspunktverschiebung - der benachbart angeordneten UV-Lampe, wie das bei den bekannten Bestrahlungsgeräten der Fall ist. Bei diesen bekannten Geräten führt

- 5 -

die Arbeitspunktverschiebung zu einer Veränderung der
abgegebenen Wellenlänge der UV-Strahlung, so daß die
Abgabe eines bestimmten, für den Behandlungserfolg
notwendigen Wellenlängenbereiches nicht möglich ist.
Erfindungsgemäß bleibt also durch Verwendung eines
sich nur geringfügig erwärmenden Kondensators der vorgewählte Arbeitspunkt der UV-Lampe auch über lange Betriebszeiten konstant, so daß ein gewünschtes Wellenlängenspektrum abgegeben wird.

Nach einer Weiterbildung der Erfindung ist vorgesehen,
daß das Gebläse ein Fön ist, auf den ein Gehäuse, in
dem sich mindestens die UV-Lampe befindet, arretierbar
aufgesteckt werden kann. Die Verwendung eines Fönes
führt zu dem Vorteil, daß einerseits der aufsteckbare
UV-Aufsatz (Gehäuse mit UV-Lampe) sehr klein ausgebildet
sein kann und andererseits - bei abgenommenem UV-Aufsatz -
der Fön als herkömmliches Gerät zum Haartrocknen verwendet
werden kann. Das erfindungsgemäße Bestrahlungsgerät
weist demnach verschiedene Betriebsarten auf, nämlich

a) UV-Bestrahlung,

b) Haartrocknung,

C) UV-Bestrahlung und Haartrocknung.

Nach einer anderen Weiterbildung der Erfindung ist
vorgesehen, daß der Kühlluftstrom des Gebläses abhängig von der Temperatur der UV-Lampe geregelt wird.
Hierdurch wird es möglich, die Temperatur der UV-Lampe
auf einer ganz bestimmten Höhe zu halten, bei der diese
eine vorgegebene UV-Strahlungsleistung und Wellenlänge
abgibt, die für die gewünschten Zwecke die optimale
Strahlung darstellt.

- 6 -

Die Verwendung eines Kondensators als Vorwiderstand
für die UV-Lampe ist für den Fachmann nicht naheliegend,
da sich bisher bei einer derartigen Anordnung schon nach
relativ kurzer Betriebszeit der UV-Lampe (wenige Minuten)
ein Flackern einstellt, auf das wenig später ein Erlöschen
der UV-Lampe folgt. Ein Dauerbetrieb ist also nach
dem bisherigen Kenntnisstand bei Verwendung eines Kondensators als Vorwiderstand nicht möglich. Der Grund für
das Erlöschen der UV-Lampe ist folgender: Mit steigender
Betriebszeit erhöht sich auch die Temperatur der UV-Lampe.
Hiermit ist ein Anstieg der Wiederzündspannung der UV-
Lampe verbunden. Bei jeder Zündung der UV-Lampe, die in
jeder Halbwelle der Netzspannung erfolgt, treten Störspannungen (Rückwirkungen) auf, deren Frequenz höher
als die der Netzspannung ist. Hierdurch erniedrigt sich
der Blindwiderstand des kapazitiven und somit frequenzabhängigen Vorwiderstandes, so daß die Spannung an der
UV-Lampe ansteigt. Dies führt jedoch zu einer Erhöhung
des Lampenstromes und gleichzeitiger Temperaturerhöhung
der UV-Lampe, was wiederum eine Erhöhung der Wiederzündspannung zur Folge hat. Gleichzeitig werden hiermit
die Störfrequenzen verstärkt, was wiederum eine weitere
Erniedrigung des Widerstandswertes des kapazitiven Vorwiderstandes bewirkt. Dieser Vorgang schaukelt sich auf,
bis die von der UV-Lampe benötigte Wiederzündspannung
größer als die zur Verfügung stehende Netzspannung ist.
Eine derartig hohe Spannung kann der UV-Lampe jedoch
nicht zur Verfügung gestellt werden, so daß diese erlischt. Die Erfindung löst das hier aufgezeigte Problem
dadurch, daß die UV-Lampe durch einen Luftstrom gekühlt
wird, wodurch ein Ansteigen der Wiederzündspannung auf
einen unzulässig hohen Wert verhindert wird, da sich

- 7 -

über die Temperatur der UV-Lampe ihre Wiederzündspannung beeinflussen läßt. Diese Maßnahme führt zu dem überraschenden Ergebnis, einen störungsfreien Dauerbetrieb mit einer UV-Lampe, der ein kapazitiver Vorwiderstand vorgeschaltet ist, durchführen zu können.

Die Zeichnungen veranschaulichen die Erfindung an mehreren Ausführungsbeispielen, und zwar zeigt

Fig. 1 einen Querschnitt durch das erfindungsgemäße Bestrahlungsgerät, teilweise geschnitten,

Fig. 2 eine Draufsicht auf das Gerät gemäß Fig. 1

Fig. 3 verschiedene Spektralverteilungen nach Reflexion der UV-Lampe an unterschiedlichen Reflektoren,

Fig. 4 eine erfindungsgemäße elektrische Schaltung einer UV-Lampe,

Fig. 5 ein Schaltbild eines Netzteils für ein Gebläse,

Fig. 6 eine Anzeigeschaltung für die Betriebstemperatur der UV-Lampe sowie für die Kühlleistung des Gebläses,

Fig. 7 ein erfindungsgemäßes Gesamtschaltbild für den Betrieb zweier UV-Lampen mit geregelter Kühlung,

Fig. 8 eine Seitenansicht eines erfindungsgemäßen Föns mit UV-Aufsatz und Abstandhalter,

- 8 -

Fig. 9   eine Draufsicht auf den Abstandhalter aus
         der Sicht des zu bestrahlenden Objektes,

Fig. 10  eine perspektivische Ansicht eines erfindungsgemäßen Föns mit abgenommenem UV-Aufsatz,

Fig. 11  eine Seitenansicht des Fönes gemäß Fig. 10
         in geöffnetem Zustand,

Fig. 12  eine erfindungsgemäße Verbindungsvorrichtung
         zwischen UV-Aufsatz und Fön,

Fig. 13  eine Draufsicht auf die Frontseite des
         UV-Aufsatzes,

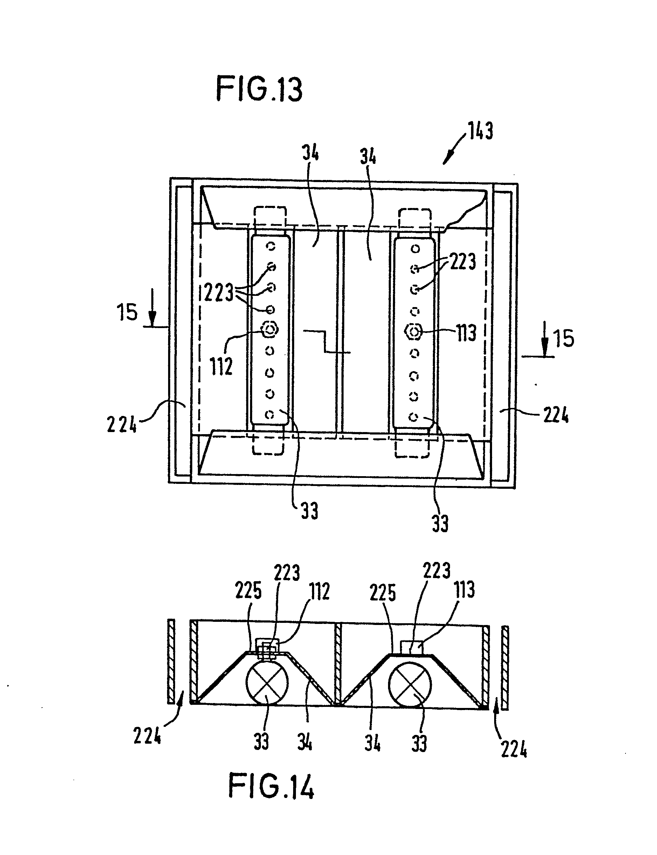

Fig. 14  eine Schnittansicht durch den Frontbereich
         des erfindungsgemäßen UV-Aufsatzes gemäß
         Fig. 13 entlang der Linie 15-15,

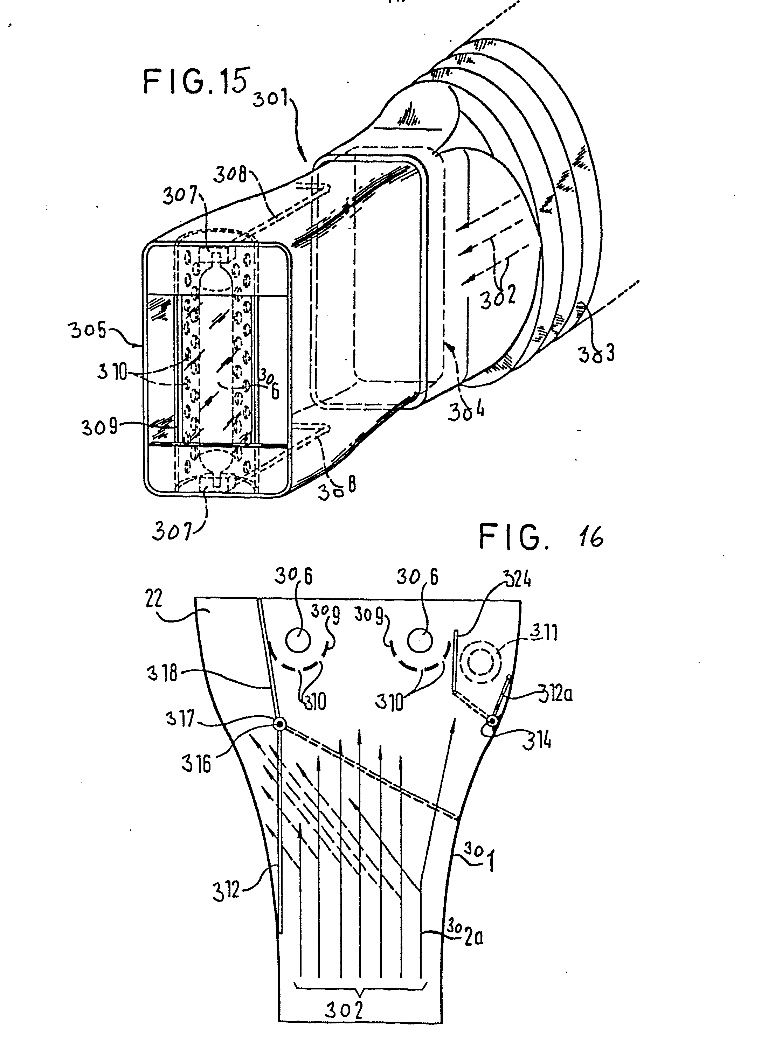

Fig. 15  eine perspektivische Ansicht einer besonders
         zweckmäßigen Bestrahlungsvorrichtung,

Fig. 16  perspektivisch eine Einsicht in eine Bestrahlungsvorrichtung nach der Erfindung.

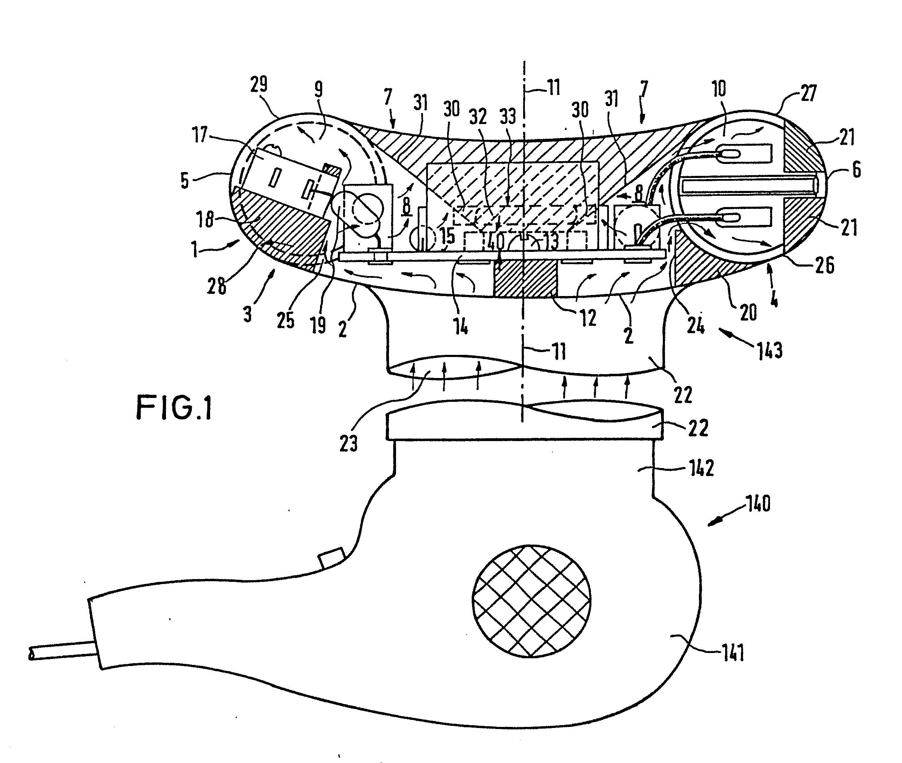

Das Gehäuse 1 des in Fig. 1 abgebildeten erfindungsgemäßen Bestrahlungsgerätes weist eine von der Bestrahlungsseite her betrachtet - das ist die in Fig. 1 oben
liegende Seite - konkave Rückwand 2 auf, die an ihren
äußeren Enden 3 und 4 in eine teilkreisförmige Abrundung 5 und 6 übergeht. Die Abrundungen 5 und 6
grenzen von der Bestrahlungsseite her gesehen an einen Reflektorraum 7. Im Innern 8 des Gehäuses 1 sind jeweils endseitig, angepaßt an die Abrundung 5 bzw. 6, Kondensatoren 9 bzw. 10 angeordnet. Mittig zur Längsachse 11 des Gehäuses 1 ist eine mit diesem verbundene Leiterplattenbefestigung 12 vorgesehen, an der mittels einer Befestigungsschraube 13 eine Leiterplatte 14 gehalten wird. Die Längsachse 11 steht senkrecht auf der Oberfläche 15 der Leiterplatte 14. Auf der Leiterplatte 14 sind verschiedene Bauteile für eine im Reflektorraum 7 angeordnete UV-Lampe 33 angeordnet. Auf der in Fig. 1 linken Seite, ist vor dem Kondensator 9 ein Mikroschalter 17 auf einer zum Gehäuse 1 gehörigen Schalterbefestigung 18 angebracht, mit dem das Bestrahlungsgerät ein- und ausgeschaltet werden kann. Das Ein- und Ausschalten erfolgt durch Druck auf einen von außen zugänglichen Druckknopf des Mikroschalters 17 (nicht dargestellt). Ein an den Mikroschalter 17 angeschlossener Thermoschalter 19 überwacht die Temperatur im Innern 8 des Gehäuses 1 und schaltet beim Überschreiten einer vorbestimmten Temperatur - z.B. 80° - das Bestrahlungsgerät aus. Jeder Kondensator 9, 10 wird von einer Halterung 20 abgestützt und von einem Verdrehschutz 21 gesichert (in Fig. 1 nur bei dem Kondensator 10 dargestellt). Vorzugsweise ist die Halterung 20 federnd ausgebildet.

An die Rückwand 2 schließt sich koaxial zur Längsachse 11 eine Aufstecktülle 22 an, die zum Aufsetzen des Gehäuses 1 auf ein Gebläse 140 dient.

- 10 -

Nach einer Weiterbildung der Erfindung ist vorgesehen, daß das Gebläse 140 ein Fön 141 ist, auf dessen Luftaustrittsstutzen 142 die Aufstecktülle 22 durch Klemmsitz befestigt werden kann. Für die Befestigung kann nach einer anderen Ausbildung auch ein grobes Schraubgewinde oder es können radial angreifende Feststellschrauben vorgesehen sein. Die von dem Gebläse 140 abgegebene Luftströmung ist in Fig. 1 durch Pfeile angedeutet, die zunächst die Öffnung 23 der Aufstecktülle 22 durchsetzt und in ihrem weiteren Verlauf durch die zwischen Leiterplatte 14 und Kondensator 9 bzw. 10 gebildeten Luftführungskanäle 24 bzw. 25 strömt. Hieran anschließend überstreicht ein Teil des Luftstromes die Kondensatoren 9 und 10 und tritt dann aus dem Gehäuse 1 durch im Bereich der Kondensatoren 9 bzw. 10 vorgesehene Auslässe 26, 27, 28 und 29 aus. Dieser Teilluftstrom umspült somit die Kondensatoren 9 und 10. Ein weiterer Teilluftstrom überstreicht die auf der Leiterplatte 14 angeordnete UV-Lampenfassung 30 und findet dann über andere, nicht dargestellte Luftauslässe seinen Weg aus dem Gehäuse 1 heraus.

- 11 -

Es ist vorgesehen, daß die den Reflektorraum 7 begrenzenden Wände 31 Öffnungen aufweisen, durch die ein Teil des Luftstromes strömt und dadurch den Glaskolben 32 der UV-Lampe 33 sowie den Reflektorraum 7 kühlt.

Es kann auch vorgesehen sein, daß im oberen Teil des Gehäuses 1 zwei Ausschnitte vorgesehen sind (nicht dargestellt), die von oben (Fig. 1) ein Einlöten der Lampenanschlußdrähte der UV-Lampe 33 in die Leiterplatte 14 ermöglichen. Die Ausschnitte werden durch das spätere Aufsetzen von Abdeckungen (nicht dargestellt) für die Lampenbefestigung durch Endkappen verdeckt. Lampenhalterung und deren Abdeckungen sind dann so ausgebildet, daß möglichst geringe Öffnungen zum Innern 8 des Gehäuses 1 verbleiben, um eine Erwärmung des Innern 8 durch die UV-Lampe 33 zu vermeiden.

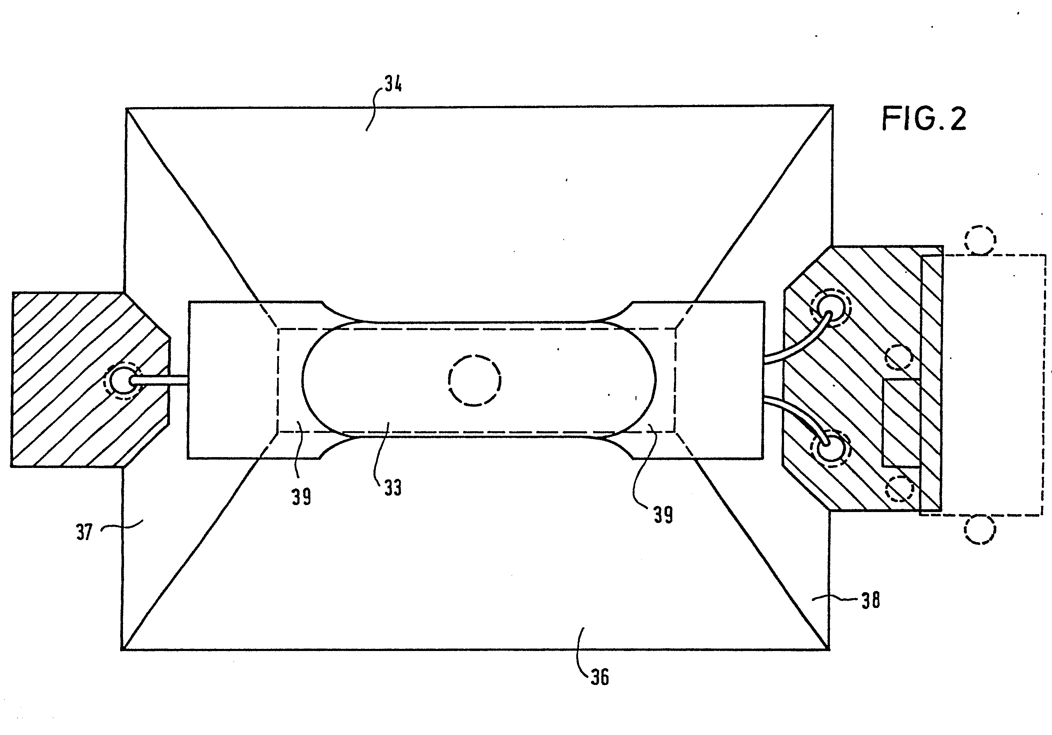

In der Fig. 2 ist eine Draufsicht auf die UV-Lampe 33 sowie deren im Reflektorraum 7 angeordneter Reflektor 34 aus der Richtung des zu bestrahlenden Objektes dargestellt. Der Reflektor 34 besteht vorzugsweise aus fünf ebenen Reflektorflächen 35 bis 39, die so angeordnet sind, daß eine optimale Reflektion der von der UV-Lampe 33 ausgehenden Strahlung erfolgt.

0104466

- 12 -

Es ist vorgesehen, daß die
Reflektorflächen 35 bis 39 mit Öffnungen versehen
sind, durch die ein Teil des von dem Gebläse erzeugten
Luftstromes zur Kühlung der UV-Lampe tritt.

Nach einer weiteren Ausführung der Erfindung können
auch mehrere UV-Lampen in dem Reflektorraum 7 angeordnet sein.

Insbesondere aus der Fig. 2 ist es ersichtlich,
daß die gesamte Reflektorfläche des Reflektors 34
zur Reflektion der UV-Strahlung zur Verfügung steht.
Dieses ist bei bekannten Bestrahlungsgeräten nicht
der Fall, denn innerhalb des Reflektorraumes ist
dort noch der als Vorwiderstand wirkende IR-Strahler
angeordnet. Hierdurch wird die effektive Reflektorfläche verkleinert, so daß die abgestrahlte Strahlungsleistung der UV-Lampe bei den bekannten Geräten
kleiner als bei dem vergleichbaren erfindungsgemäßen
Gerät ist.

Durch die Ausbildung des Reflektors 34 mit seinen
fünf Reflektorflächen 35 bis 39 wird durch die erfindungsgemäße Vorrichtung das Bestrahlungsobjekt
aus mehreren Richtungen mit divergierendem Licht
bestrahlt, so daß auch nicht ebene Teile oder Flächen
des Bestrahlungsobjektes, z. B. die Gesichtsfläche
einer zu bestrahlenden Person, gleichmäßig bestrahlt
werden. Unabhängig von dem unterschiedlichen Abstand
zum Gerät erhält jede Stelle des Körpers eine nahezu gleiche

- 13 -

Strahlungsdosis. Darüber hinaus ist der erfindungsgemäße Reflektor mit seinen ebenen Flächen gegenüber
dem bei bekannten Vorrichtungen eingesetzten parabolförmigen Reflektor kostengünstiger und einfacher herzustellen.

Nach einer Weiterbildung der Erfindung ist vorgesehen,
daß zur Unterdrückung bestimmter Spektralbereiche
der UV-Bestrahlung und/oder langwelliger sichtbarer
Strahlung und/oder IR-Strahlung zusätzlich
im Reflektorraum 7 angeordnete Filtervorrichtungen
vorhanden sind. Da die Spektralverteilung der auf
den zu bestrahlenden Körper auftreffenden Strahlung
auch im hohen Maße vom Material der Oberflächenschicht
des Reflektors beeinflußt wird, wird dafür vorzugsweise ein Material verwendet, daß UV-A-Strahlung
gut reflektiert. Derartige Materialien sind z. B.
gelb eloxiertes Aluminium, hochglanzpolierter Edelstahl mit hohem Chrom-Anteil oder Chrom.

Um sich eine Vorstellung über die Abmessungen des
beschriebenen Ausführungsbeispiels machen zu können,
sollen einige Maße angegeben werden. Die Radien
der Abrundungen 5 und 6 betragen etwa 140 mm; der
Abstand zwischen der Unterseite der Leiterplatte
14 und der Gehäuserückwand 2 beträgt im Bereich
der Längsachse 11 etwa 6 mm. Der Abstand zwischen
der Oberfläche 15 der Leiterplatine 14 und dem Reflektor 34 sollte zweckmäßigerweise wegen der Befestigungsschraube 13 und aus verdrahtungstechnischen
Gründen einen in der Fig. 1 mit 40 bezeichneten

- 14 -

Abstand von etwa 3 mm aufweisen. Das erfindungsgemäße Bestrahlungsgerät wiegt insgesamt nur etwa 250 - 300 g, ist kaum größer als eine Zigarettenschachtel und kann deshalb leicht mitgeführt werden.

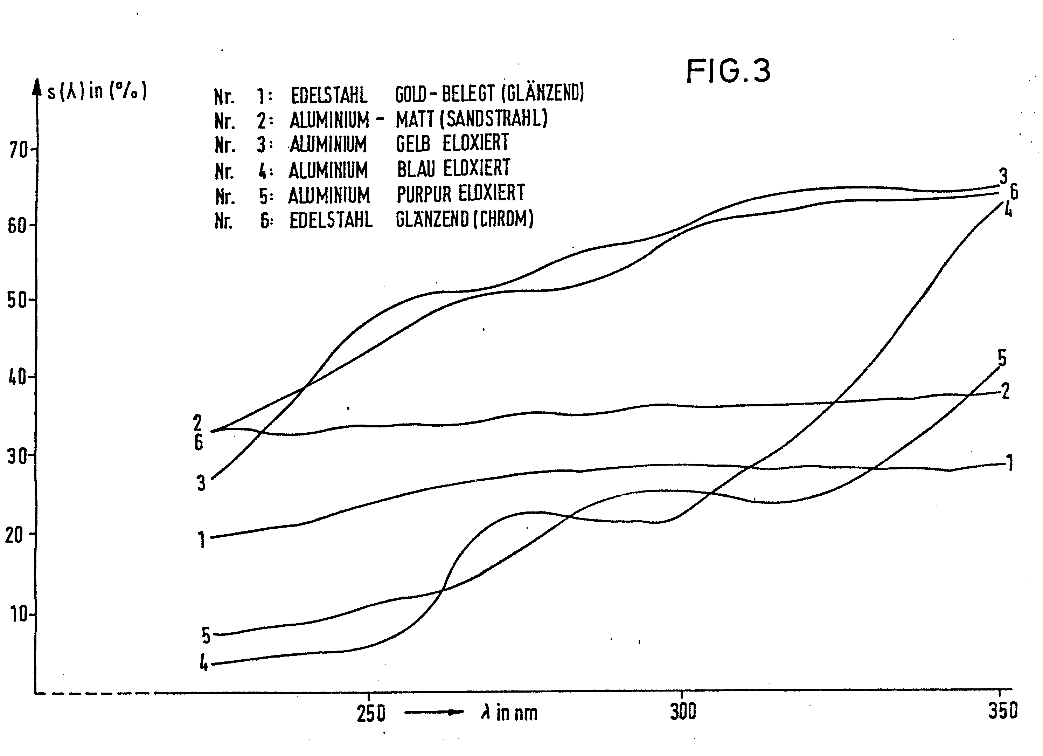

Die Fig. 3 zeigt die Spektralverteilung der in Richtung auf den zu bestrahlenden Körper emittierten UV-Strahlung für verschiedene Oberflächenschichten des Reflektors 34, wobei die Oberflächenschichten 3 (Aluminium gelb eloxiert) und 6 (Edelstahl glänzend (Chrom)) besonders bevorzugt sind.

Für die Zuführung von elektrischer Energie zu der UV-Lampe 33 ist am Fön 141 eine Buchse angeordnet, in die ein an der Aufstecktülle 22 angeordnetes Steckerteil beim Aufsetzen des Gehäuses 1 auf den Fön 141 eingreift.

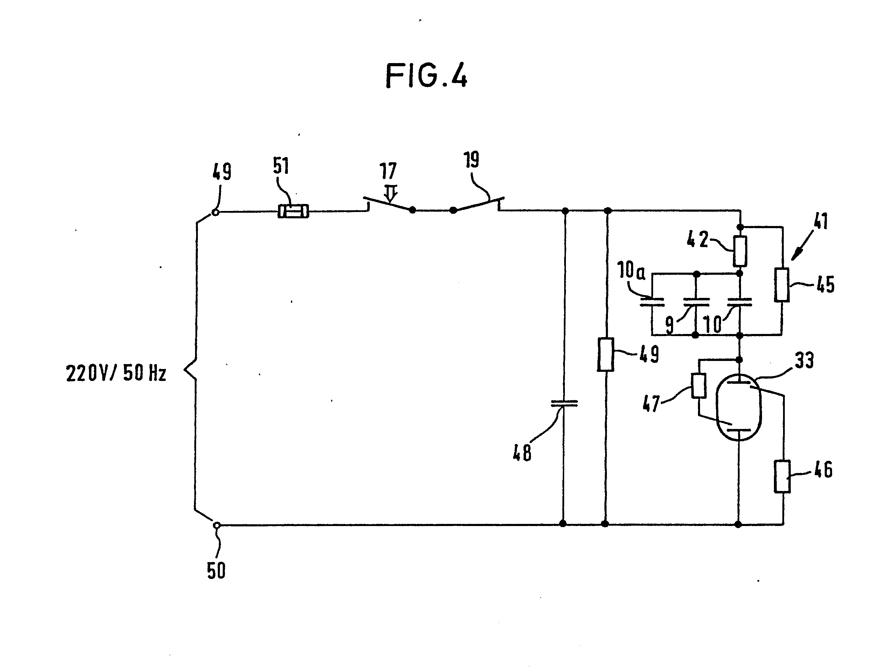

Die Fig. 4 zeigt die elektrische Schaltung der UV-Lampe 33. In Reihe zu der UV-Lampe 33 liegt ein aus ohm'schen und kapazitiven Widerständen zusammengesetzter Vorwiderstand 41. Der Vorwiderstand 41 besteht aus einem Leistungswiderstand 42, zu dem in Reihe drei parallel geschaltete Kondensatoren 9, 10 und 10a liegen. Parallel zu der aus dem Widerstand 42 und den Kondensatoren 9, 10 und 10a gebildeten Reihenschaltung liegt ein Widerstand 45. Bei Verwendung einer Quecksilberdampf-Hochdrucklampe OS 124 (Nennleistungsaufnahme 125 W, Brennerspannung 85 V $\pm$ 11 V bei einem Nennstrom von ca. 2A; Hersteller Osram GmbH) als UV-Lampe 33 wird ein kapazitiver Vorwiderstand bei Netzbetrieb (220 V / 50 Hz) von

- 15 -

30 µF benötigt. Somit besitzen die Kondensatoren 9, 10 und 10a jeweils eine Kapazität von 10 µF. Der Widerstand 42 hat einen Wert von 0,5 Ohm und dient zur Spitzenstrombegrenzung. Der Widerstand 45 dient zum Entladen der Kapazitäten nach Ausschaltung des Gerätes und hat einen Wert von etwa 100 k$\Omega$ . Gezündet wird die vorliegende UV-Lampe über die Widerstände 46 und 47.

Parallel zu der Reihenschaltung aus Vorwiderstand 41 und UV-Lampe 33 liegen eine Kapazität 48 sowie ein Widerstand 49. Diese Bauelemente dienen der Entstörung. Die Zuführung der Versorgungsspannung über die Klemmen 49 und 50 erfolgt über die Reihenschaltung einer Sicherung (Strombegrenzung) 51, dem Mikroschalter 17 und dem Thermoschalter 19.

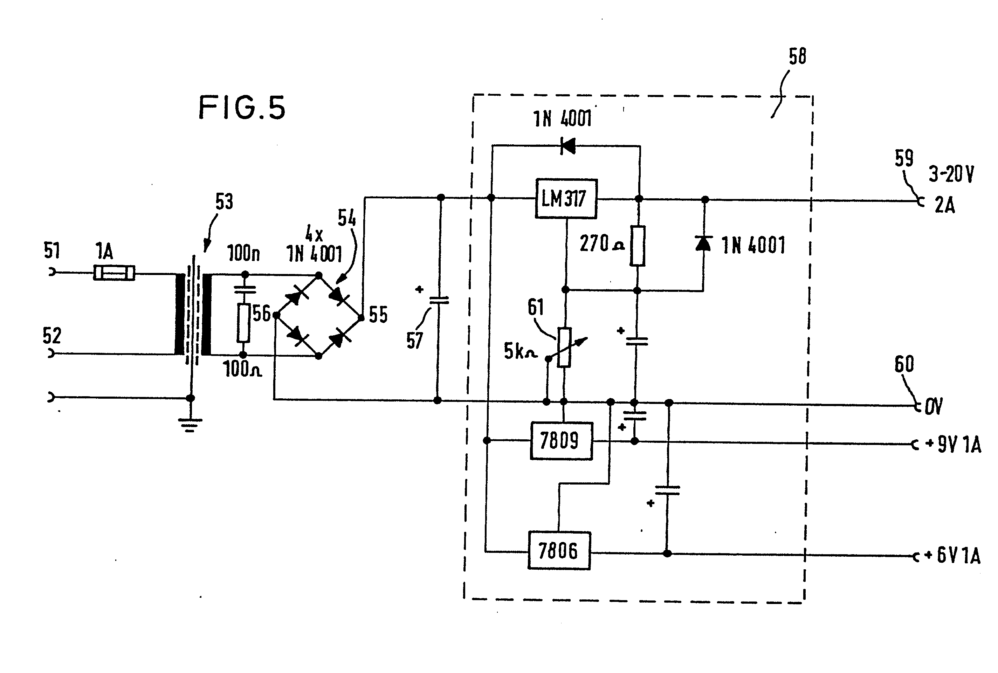

In der Fig. 5 ist ein Netzgerät dargestellt, das zur Versorgung des Antriebsmotors (Gleichstrommotor) des Fönes 141 dient. An die Eingangsklemmen 51 und 52 wird Netzspannung (220V / 50 Hz) gelegt, die mittels des Transformators 53 heruntergespannt und dem Brückengleichrichter 54 zugeführt wird. An den Ausgängen 55 und 56 des Brückengleichrichters 54 liegt dann eine Gleichspannung an, die von dem Kondensator 57 geglättet und einer Regel- und Stabilisierungsschaltung 58 zugeführt wird. die an den Ausgangsklemmen 59 und 60 anliegende Ausgangsspannung läßt sich mit Hilfe des Potentiometers 61 regeln. Da an die Ausgangsklemmen 59 und 60 der Gleichstrommotor des Fönes angeschlossen wird, ist dessen Drehzahl durch Verstellung des Potentiometers 61 ein-

- 16 -

stellbar, wodurch die aus dem Fön 141 austretende Luftmenge gesteuert werden kann. Mit Änderung der Betriebstemperatur der UV-Lampe 33 variiert sowohl ihre Leistung als auch die Wellenlänge der abgegebenen Strahlung. An dem Potentiometer 61 läßt sich - wie schon
beschrieben - die Luftmenge und damit die Kühlwirkung
der UV-Lampe 33 steuern. Hiermit ist also die Möglichkeit geschaffen, sowohl auf die Leistung als auch auf
die Wellenlänge der UV-Lampe 33 Einfluß zu nehmen.
Je nach dem vorliegenden Bedürfnis kann das erfindungsgemäße Bestrahlungsgerät damit optimal eingestellt werden.

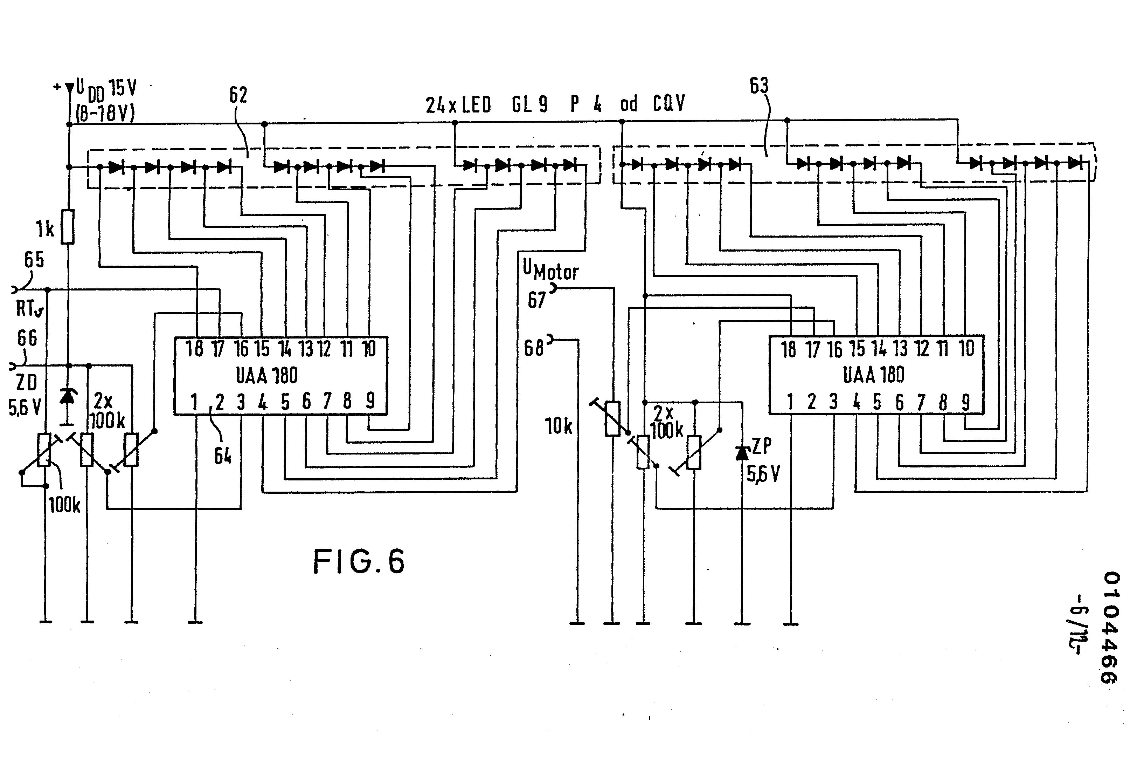

Die Fig. 6 zeigt eine Anzeigeschaltung, die zwei
Leuchtdioden-Reihen 62 und 63 aufweist. Die Leuchtdiodenreihe 62 wird durch den integrierten Schaltkreis 64 (UAA 180) angesteuert und dient der Anzeige
der in dem erfindungsgemäßen Bestrahlungsgerät vorherrschenden Temperatur. An die Klemmen 65 und 66
wird hierzu eine der Temperatur im Innern 8 des
Gehäuses 1 proportionale Spannung angelegt, die
in dem integrierten Schaltkreis 64 ausgewertet wird
und eine entsprechende Anzahl von Leuchtdioden der
Leuchtdioden-Reihe 62 zum Ansprechen bringt. Als
Temperaturfühler kann beispielsweise ein NTC-Widerstand verwendet werden. Der NTC-Widerstand kann
direkt oder indirekt mit dem Glaskolben der
UV-Lampe 33 in Kontakt stehen, so daß die Anzahl
der aufleuchtenden Leuchtdioden ein Maß für die
Kolbentemperatur und somit für die abgegebene Strahlungsleistung und für die Wellenlänge der von der
UV-Lampe 33 abgegebenen Strahlung ist. Die Anzeigeschaltung kann dabei so ausgeführt sein, daß sich
bei steigender Temperatur die Anzahl der leuchtenden Leuchtdioden erhöht.

- 17 -

Die andere Leuchtdioden-Reihe 63 arbeitet in analoger Weise; ihr wird jedoch über die Klemmen 67 und 68 die Motorspannung des Föns 141 zugeführt, so daß die Anzahl der leuchtenden Leuchtdioden der Leuchtdioden-Reihe 63 ein Maß für die abgegebene Kühlluftmenge ist. Der Benutzer des Gerätes kann somit über die beiden Leuchtdiodenanzeigen einen gewünschten Arbeitspunkt der erfindungsgemäßen Vorrichtung einstellen.

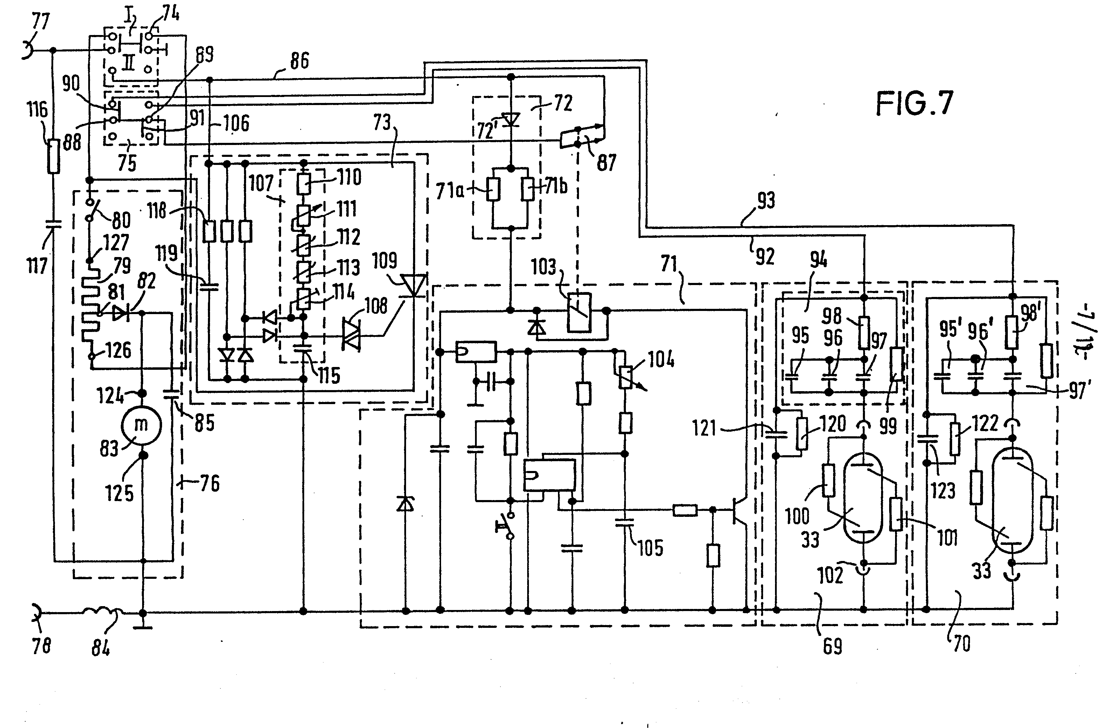

Die Fig. 7 zeigt ein Gesamtschaltbild für ein erfindungsgemäßes Bestrahlungsgerät, wobei zwei UV-Lampen und eine automatische Kühlluftregelung vorgesehen sind. Die Gesamtschaltung setzt sich aus zwei UV-Lampenschaltungen 69 und 70, einer Zeitschaltung 71, einer Versorgungsschaltung 72 für die Zeitschaltung 71, einer Phasenanschnittschaltung 73, einem Fönschalter 74, einem UV-Lampenschalter 75 und einer Fönschaltung 76 zusammen.

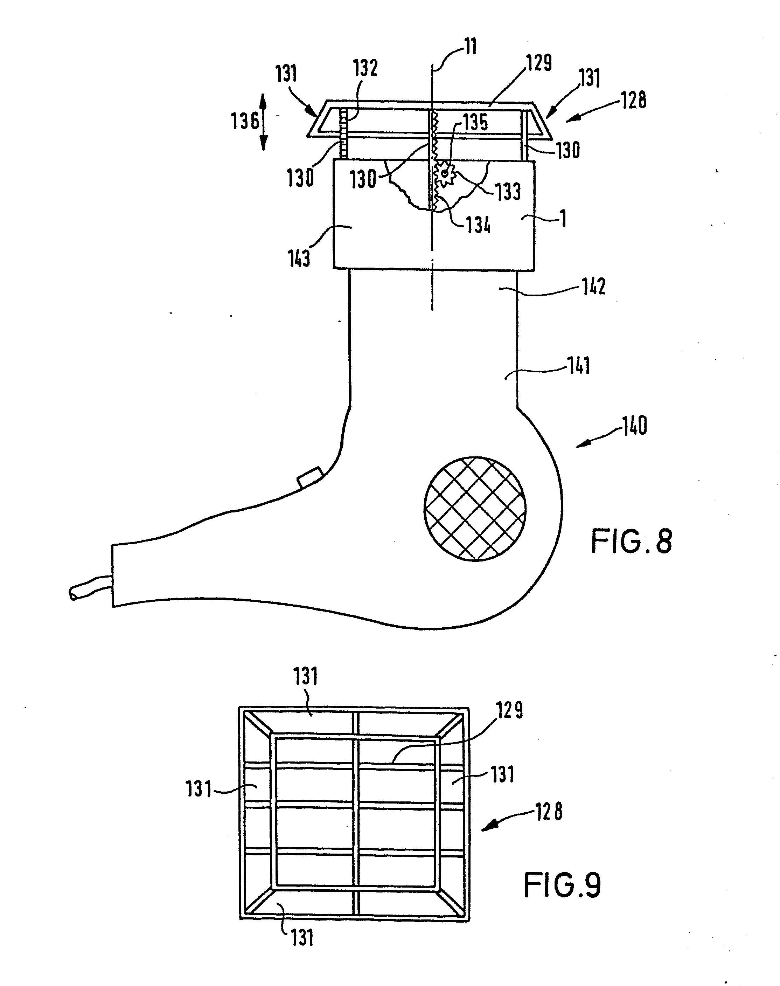

Wird an die beiden Klemmen 77 und 78 Netzspannung angelegt (220 V / 50 Hz) und befindet sich der Fönschalter 74 in der in Fig. 8 eingezeichneten Stellung I, so wird die Heizung 79 des Föns in Betrieb gesetzt. Die Heizung 79 besteht aus Widerstanddraht, der sich beim Stromdurchfluß erwärmt. In Reihe zur Heizung 79 liegt ein Temperaturschalter 80, der beim Überschreiten einer vorgegebenen Temperatur die Heizung 79 ausschaltet. Die Heizwicklung der Heizung 79 besitzt einen Abgriff 81, an dem eine Diode 82 angeschlossen ist. Die Diode ist mit dem einen Pol 124 eines Gleichstrommotors 83 verbunden; dessen anderer

- 18 -

Pol 125 über die Drossel 84 mit der Klemme 78 in Verbindung steht. Bei dem Gleichstrommotor 83 (z.B. ein 12 V-
Motor) handelt es sich um den Gebläsemotor des Föns.
Parallel zu dem Gleichstrommotor 83 liegt ein Kondensator 85, der die Motorspannung glättet. In diesem
Betriebszustand (Fönschalter 74 in Stellung I) ist
der Fön in herkömmlicher Art und Weise zu benutzen.

Wird der Fönschalter 74 in seine Stellung II
gebracht, so wird über die Leitung 86 bei geschlossenem
Relaiskontakt 87, die Anschlüsse 88 und 89 des UV-Lampenschalter 75 an Spannung gelegt. Je nach Stellung
der Schaltkontakte 90 und 91 können die UV-Lampenschaltungen 69 und 70 über die Leitungen 92 und
93 ein- bzw. ausgeschaltet werden. Die UV-Lampenschaltung 69 besteht aus einem Vorwiderstand 94,
der drei parallel geschaltete Kondensatoren 95 bis
97 (jeweils 10 µF), einem dazu in Reihe liegenden
Leistungswiderstand 98 (0,5 $\Omega$) und einen parallel
zu der Reihenschaltung von Leistungswiderstand 98
und Kondensator 97 liegenden Widerstand 99 (100 k$\Omega$)
umfaßt. In Reihe zu dem Vorwiderstand 94
liegt die UV-Lampe 33, deren Zündelektroden jeweils
mit Widerständen 100 und 101 verbunden sind. Die
Widerstände 100 und 101 besitzen einen Wert von
15 k$\Omega$ und dienen der Erzeugung der Zündspannung.
Der in Fig. 7 untere Anschluß 102 ist über die
Drossel 84 mit der Klemme 78 verbunden. Die drei
Kondensatoren 95, 96 und 97 dienen somit als Vorwiderstand für die UV-Lampe 33. Der Widerstand 98 begrenzt
den Spitzenstrom, und der Widerstand 99 dient der

0104466

- 19 -

Kondensatorentladung nach dem Abschalten der UV-Lampenschaltung 69. Die UV-Lampenschaltung 70 ist ebenso wie
die UV-Lampenschaltung 69 aufgebaut.

Die beiden UV-Lampenschaltungen 69 und 70 können nur in
Betrieb gesetzt werden, wenn der Relaiskontakt 87 geschlossen ist. Dieser Relaiskontakt gehört zu einem Relais 103
der Zeitschaltung 71. Zeitbestimmende Glieder der Zeitschaltung 71 sind das Potentiometer 104 und der Kondensator 105. An dem Potentiometer 104 kann die Anzugszeit
des Relais 103 eingestellt werden. Hiermit läßt sich also
die Einschaltdauer der UV-Lampenschaltungen 69 und 70
bestimmen. Da Zeitschaltungen aus dem Stand der Technik
bekannt sind, soll auf die nähere Ausführung der Zeitschaltung 71 nicht eingegangen werden. Die
Versorgungsschaltung 72 für die Zeitschaltung 71
besteht aus einer Diode 72'
und zwei parallel geschalteten Leistungswiderständen
71a,b. Diese sind so angeordnet, daß sie durch den Luftstrom des Fönes gekühlt werden, so daß die erzeugte
Wärme abgeführt werden kann.

Befindet sich der Fönschalter 74 in seiner Stellung II,
so wird über die Leitung 106 die Phasenanschnittschaltung 73 mit Spannung versorgt. Die Phasenanschnittschaltung 73 weist ein anschnittswinkelbestimmendes
Glied 107 auf, daß ein Diac 108 ansteuert, welches
mit dem Gate eines Tyristors 109 in Verbindung steht.
Der Tyristor 109 liegt in der beschriebenen Betriebsstellung des Fönschalters 74 in Reihe mit der Fönschaltung 76, wobei die Reihenschaltung von Tyristor 109 und Fönschaltung 76 über die Drossel 84 an Betriebsspannung
(220 V / 50 Hz) liegt. Mit Variation des Phasenanschnittswinkels der Phasenanschnittschaltung 73 läßt sich die
Versorgungsspannung des Fönes variieren, wodurch die
Motordrehzahl des Gleichstrommotors 83 veränderbar
ist. Die Versorgungsspannung liegt in dieser Betriebsart an der aus einem Teilstück der Heizung 79 (vom
Anschluß 120 der Heizwicklung bis zum Abgriff 81), der
Diode 82 und dem Motor 83 gebildeten Reihenschaltung
an.

Das den phasenanschnittwinkelbestimmende Glied 107 der Phasenanschnittsschaltung 73 wird durch die Reihenschaltung
von Widerstand 110 Potentiometer 111, NTC-Widerstand 112, NTC-Widerstand 113, Trimmer 114 und Kondensator 115 gebildet. Die beiden NTC-Widerstände
112 und 113 sind jeweils einer der UV-Lampenschaltungen 69 bzw. 70 zugeordnet, d. h. jeder der beiden
Widerstände befindet sich in unmittelbarer Nähe
der entsprechenden UV-Lampe, so daß die von der
jeweiligen UV-Lampe ausgehende Wärme von dem entsprechenden Widerstand registriert wird. Je nach
Wärmebeaufschlagung der NTC-Widerstände verändern
diese ihren Widerstandswert, wodurch der Phasenanschnittswinkel der Phasenanschnittsschaltung 73
verändert und damit auf die Motordrehzahl des Gleichstrommotors 83 Einfluß genommen wird. Mit Veränderung
der Motordrehzahl ändert sich die von dem Fön abgegebene Kühlluftmenge, die bei der erfindungsgemäßen
Vorrichtung zur Kühlung der UV-Lampe 33 verwendet
wird. Im Nachfolgenden wird nur von einer UV-Lampe
gesprochen, es können natürlich auch mehrere - so wie in der Fig. 7 z. B. zwei - UV-Lampen verwendet werden. Zur Beschreibung des Regelkreises soll davon ausgegangen werden, daß die Temperatur der UV-Lampe 33 der UV-Lampenschaltung 69 steigt. Hierdurch verändert auch der in unmittelbarer Nachbarschaft liegende NTC-Widerstand 112 seine Temperatur und damit seinen Widerstandswert, wodurch sich der Phasenanschnittswinkel der Phasenanschnittsschaltung 73 derart ändert, daß der Fönschaltung 76 eine höhere Spannung zugeführt wird. Damit erhöht sich auch die Drehzahl des Gleichstrommotors 83, wodurch eine größere Luftmenge aus dem Fön 141 ausgestoßen wird, die wiederum zur intensiveren Kühlung der UV-Lampe 33 führt, so daß eine bestimmte Temperatur an der UV-Lampe 33 erreicht wird. Erniedrigt sich nun die Temperatur an der UV-Lampe 33, so wirkt die Regelschaltung im entgegengesetzten, analogen Sinne. Der NTC-Widerstand 113 ist der UV-Lampenschaltung 70 zugeordnet. Der Arbeitspunkt der Strahler kann an dem Trimmer 114 der Motorregelung genau justiert werden. Mit Hilfe des Potentiometers 111 läßt sich manuell auf die Regelschaltung Einfluß nehmen.

Da in der in Fig. 7 dargestellten erfindungsgemäßen Schaltung an verschiedenen Stellen starke Störspannungen auftreten, sind vier R-C-Kombinationen (116, 117; 118, 119; 120, 121; 122, 123) sowie die Drossel 84 zur Entstörung vorgesehen.

Im automatischen Regelbetrieb des erfindungsgemäßen Bestrahlungsgerätes ist die Wärmeabgabe der Heizung 79 des Fönes nicht von Bedeutung; d.h. die mit der Regelung einhergehende unterschiedliche Wärmeabgabe der Heizung 79 macht sich nicht störend bei der Aufrechterhaltung eines ganz bestimmten voreingestellten Betriebszustandes bemerkbar. Mit Hilfe der in Fig. 7 dargestellten Regelschaltung läßt sich die Temperatur der UV-Lampe 33 konstant halten bzw. auf einen beliebigen Wert einstellen, der dann konstant beibehalten wird. Da sowohl die Strahlungsleistung als auch die abgegebene Wellenlänge der Strahlung der UV-Lampe 33 von ihrer Betriebstemperatur abhängig ist, läßt sich durch Regelung der Betriebstemperatur die gewünschte Strahlungsleistung und auch Wellenlänge einstellen. Mit Hilfe des erfindungsgemäßen Behandlungsgerätes läßt sich also der für jeden Behandlungszweck optimale Arbeitspunkt der UV-Lampe 33 einstellen. Darüber hinaus ermöglicht die geregelte Kühlung, daß das erfindungsgemäße Bestrahlungsgerät im Langzeitbetrieb eingesetzt werden kann.

Nach einem weiteren Ausführungsbeispiel der Erfindung ist vorgesehen, daß an dem Gehäuse 1 des UV-Aufsatzes 143 ein Abstandshalter 128 befestigt ist (Fig. 8 ). Der Abstandshalter 128 besteht aus einem ebenen Gitter 129 aus Kunststoff oder Draht, das etwa die Größe der dem Bestrahlungsobjekt zugewandten Seite des UV-Aufsatzes 143 besitzt. An dem Gitter 129 sind einendig Abstandstreben 130 befestigt, die rechtwinklig zu der Gitterebene verlaufen und anderendig in Richtung der Längsachse 11 verschieblich sowie arretierbar an dem Gehäuse 1 geführt sind. Die Randbereiche 131 des Gitters 129 sind in Richtung auf das Gehäuse 1 abgewinkelt. Durch die Längsverschieblichkeit kann das Gitter auf einen vorgegebenen Abstand zur UV-Lampe 33 des UV-Aufsatzes 143 eingestellt werden. Zur genauen Ermittlung des Abstandes kann ggfs.

- 23 -

eine an einer Abstandstrebe 130 angeordnete Stricheinteilung 132 zu Hilfe genommen werden. Ein an dem Gehäuse 1 drehbar gelagertes Zahnrad 133 kämmt mit einer Zahnung 134 einer der Abstandstreben 130. Das Zahnrad ist einstückig sowie koaxial mit einem Bedienknopf 135 verbunden, der außerhalb des Gehäuses 1 angeordnet ist. Durch manuelle Drehung des Bedienknopfes 135 läßt sich somit der Abstandshalter 128 in Richtung des in Fig. 9 eingetragenen Doppelpfeils 136 verfahren. Mit Hilfe des Abstandshalters 128 kann der Anwender des erfindungsgemäßen Bestrahlungsgerätes die Entfernung zwischen Behandlungsstelle (z. B. Haut) und UV-Lampe 33 genau einhalten. Hierzu wird das Gitter 129 gegen die zu bestrahlende Behandlungsstelle gedrückt. Gerade an schlecht zugänglichen Behandlungsstellen, z. B. Behandlung der Kopfhaut im Hinterkopfbereich, erweist sich der Abstandhalter 128 als besonders hilfreich, da er durch Wahrung des genauen Bestrahlungsabstandes eine optimale Behandlung ermöglicht.

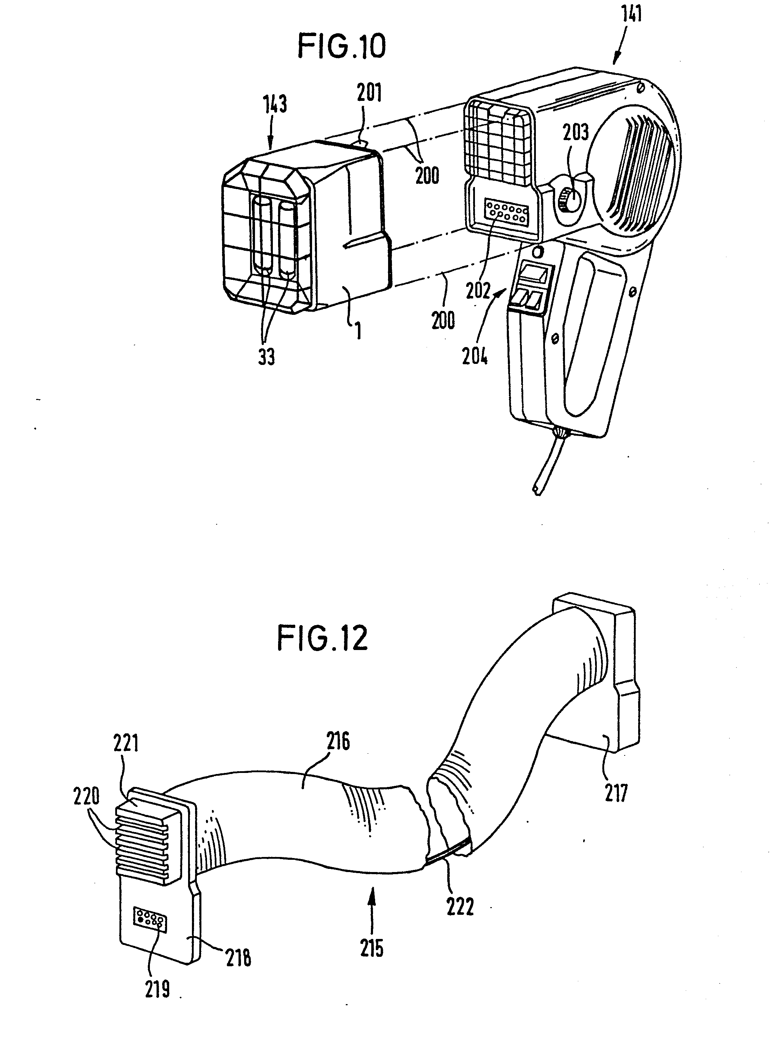

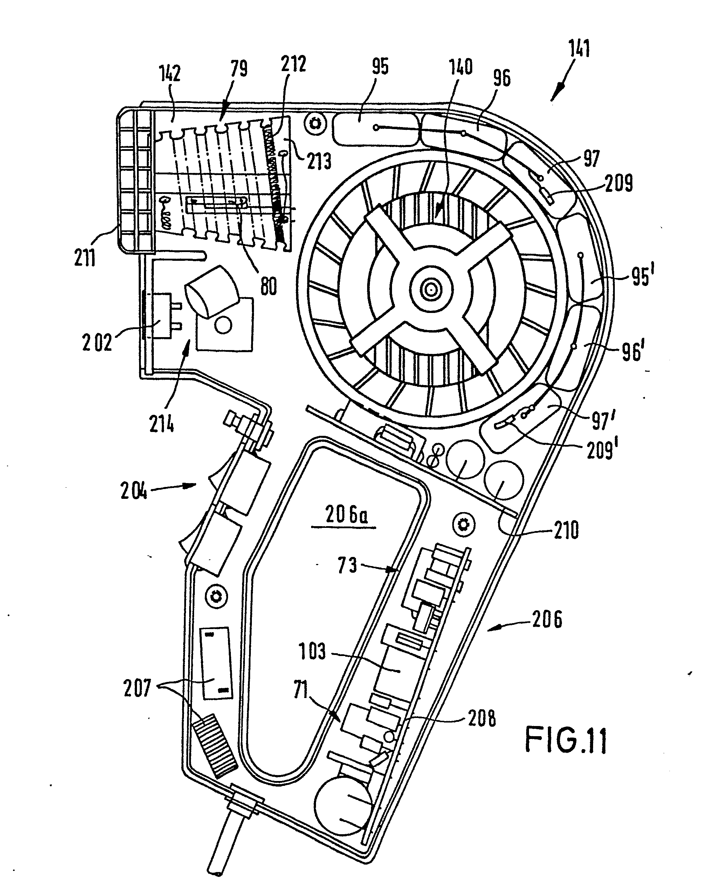

In der Fig. 10 ist ein weiteres Aufführungsbeispiel des erfindungsgemäßen Bestrahlungsgerätes gezeigt. Hierbei sind Haarfön 141 sowie UV-Aufsatz 143 voneinander getrennt. Der UV-Aufsatz 143 kann entlang der gestrichelt gezeichneten Geraden 200 in Richtung auf den Haarfön 141 bewegt werden, bis beide Teile mittels einer Rastfeder 201, die sich, in der Fig. 10 gesehen, im oberen Bereich des UV-Aufsatzes 143 befindet, verrasten. In der verrasteten Stellung werden die notwendigen elektrischen Verbindungen zwischen dem Haarfön 141

- 24 -

und dem UV-Aufsatz 143 mittels einer am Haarfön 141 angeordneten Buchse 202 und einem entsprechenden, nicht dargestellten Steckerteil an dem UV-Aufsatz 143 hergestellt. Ein seitlich an dem Haarfön 141 angebrachter Drehknopf 203 dient zum Einstellen der Bestrahlungszeit; ist also mit dem Potentiometer 104 der in der Fig. 7 dargestellten Zeitschaltung 7 verbunden. Die insgesamt mit 204 bezeichneten Bedienelemente stellen einen Schalter für das Gebläse des Fönes 141, Schalter für die im Gehäuse 1 des UV-Aufsatzes 143 angeordneten UV-Lampen 33 sowie einen Taster zum Starten der Zeitschaltung 71 (Fig. 7) dar.

In der Fig. 11 ist der Fön 141 der Fig. 10 in geöffnetem Zustand dargestellt. In das Föngehäuse 205 ist ein geschlossener, bügelförmiger Handgriff 206 integriert. Dieser Handgriff weist, in der Fig. 11 betrachtet, auf der linken oberen Seite die Bedienelemente 204 auf. Darunter sind Bauelemente 207 angeordnet, die der Entstörung der erfindungsgemäßen, in Fig. 7 dargestellten Schaltung dienen. In die rechte Seite des Handgriffs 206 ist eine mit Bauelementen bestückte Platine 208 eingesetzt, die in ihrem unteren Bereich die Zeitschaltung 71, daran nach oben anschließend das Relais 103 und im oberen Bereich die Phasenanschnittschaltung 73 aufweist. Im Kopfbereich des Fönes 141 ist das Gebläse 140 angeordnet, das halbkreisförmig von den Kondensatoren 95, 96 und 97 umgeben wird. Diese Kondensatoren gehören zu dem Vorwiderstand 94 der UV-Lampenschaltung 69. Die weiteren drei an die vorgenannten Kondensatoren anschliessenden Kondensatoren 95', 96' und 97' gehören zu dem

- 25 -

Vorwiderstand der zweiten UV-Lampenschaltung 70. Weitere, zu den Vorwiderständen gehörende Bauelemente (Widerstände) sind durch die Bezugsziffern 209 sowie 209' angedeutet. Unterhalb des Gebläses 140 sind auf der Platine 210 die Leistungswiderstände 98 bzw. 98' der Vorwiderstände sowie weitere Entstörglieder der erfindungsgemäßen Schaltung angeordnet.

Der Luftaustrittstutzen 142 des Fönes 141 ist mittels eines Gitters 201 abgedeckt, so daß ein unbeabsichtigtes Berühren der dort angeordneten Heizung 79 verhindert wird. Die Heizung besteht aus in sich gewendeltem Widerstandsdraht 212, der auf einen temperaturbeständigen Wickelkörper 213 aufgewickelt ist. An dem Wickelkörper 213 ist der schon aus der Fig. 7 bekannte Temperaturschalter 80 angeordnet.

Unterhalb des Luftaustrittstutzens 142 ist die Buchse 202 befestigt; des weiteren befinden sich hier die Bauteile 214, die der Motorsiebung dienen.

Nach einem weiteren Ausführungsbeispiel der Erfindung ist vorgesehen, den UV-Aufsatz mittels einer in Fig. 12 dargestellten flexiblen Verbindungsvorrichtung 215 an dem Fön zu befestigen. Die Verbindungsvorrichtung 215 besteht aus einem flexiblen Schlauch 216, der an seinem einen Ende mit einer Tülle 217 verbunden ist, die auf den Luftaustrittstutzen des Fönes (nicht dargestellt) aufsteckbar ist. An seinem anderen Ende ist eine Platte 218 befestigt, die, in der Fig. 12 betrachtet, in ihrem unteren Bereich eine Buchse 219 für den UV-Aufsatz 143

- 26 -

aufweist, der auf einen mit Luftaustrittsschlitzen 220 versehenen Ansatz 221, der mit dem Schlauch 216 in Verbindung steht, aufsetzbar ist. Die Buchse 219 ist über ein in dem flexiblen Schlauch 216 verlaufenden Versorgungskabel 222 und weiteren nicht dargestellten Verbindungselementen mit dem nicht dargestellten Fön verbunden. Diese Verbindungsvorrichtung 215 ermöglicht es, den Fön 141 stationär in einem Halter od.dgl. anzuordnen und mittels des auf die Verbindungsvorrichtung 215 aufgesetzten UV-Aufsatzes 143 die Bestrahlungsbehandlung vorzunehmen. Der Anwender braucht hierbei nur den relativ leichten, an der Verbindungsvorrichtung 215 angeschlossenen UV-Aufsatz 143 halten; darüber hinaus ermöglicht der flexible Schlauch 116 auch eine Behandlung an schlecht zugänglichen Körperstellen.

In der Fig. 13 ist ein weiteres Ausführungsbeispiel eines erfindungsgemäßen UV-Aufsatzes 143 von der Bestrahlungsseite her gezeigt. Die Front dieses UV-Aufsatzes 143 weist zwei nebeneinander angeordnete Reflektoren 34 auf, in deren Mitte sich jeweils eine UV-Lampe 33 befindet. Unterhalb der UV-Lampen 33 sind Luftaustrittsöffnungen 223 angeordnet, durch die ein Teil des von dem Fön erzeugten Luftstromes strömt und somit die UV-Lampen 33 kühlt. Die Luftaustrittsöffnungen 223 erstrecken sich jeweils über die gesamte Länge der UV-Lampen 33 und sind auf einer geraden Linie mit gleichmäßigem Abstand voneinander angeordnet. Beidseitig der Front des UV-Aufsatzes 143 befindet sich jeweils ein parallel zu den UV-Lampen 33 verlaufender Luftaustrittsschacht 224. Bei dem Betrieb des erfindungsgemäßen Strahlungsgerätes

- 27 -

·strömt aus diesen beiden Luftaustrittsschächten 224
ein Teil des von dem Fön erzeugten Luftstromes aus,
der dann von dem Anwender auf die Behandlungsstelle
geleitet wird.

In der Fig. 14 ist ein Schnitt durch den Frontbereich
des UV-Aufsatzes 143 gemäß der Fig. 13 entlang der
Linie 15-15 gezeigt. Nochmals sind hier die Luftaustrittsöffnungen 223 eingezeichnet, die mit der jeweiligen
UV-Lampe 33 fluchten. Auf der der jeweiligen UV-Lampe 33
abgewandten Seite /225 des jeweiligen Reflektors 34 sind
in unmittelbarer Nähe der Luftaustrittsöffnungen die
beiden aus der Fig. 7 bekannten NTC-Widerstände 112
bzw. 113 angeordnet. Diese NTC-Widerstände dienen der
Ermittlung der UV-Lampen-Temperatur.

Die in den Fig. 10, 11, 13 und 14 dargestellten Ausführungsbeispiele des erfindungsgemäßen Bestrahlungsgerätes
weisen jeweils zwei UV-Lampen 33 auf. Hierbei ist es
zweckmäßig, unterschiedliche UV-Lampen zu verwenden;
so kann z.B. einmal eine UV- A -Strahlung und zum anderen
eine UV-B-A-Strahlung abgebende Lampe verwendet werden.
Nach einem anderen Ausführungsbeispiel ist es jedoch
auch denkbar, daß die in den Fig. 10, 11, 13 und 14
dargestellten Gegenstände jeweils nur·eine·UV-Lampe
aufweisen.

- 28 -

In den dargestellten Ausführungsbeispielen ist der UV-Strahler jeweils in einem separaten, auf den Luftaustrittsstutzen aufsetzbaren Teil angeordnet. Die Erfindung bezieht sich jedoch auch auf die Anordnung des UV-Strahlers unmittelbar im Föngehäuse, und zwar im Bereich des Luftaustrittsstutzens, wobei im übrigen die elektrische Schaltung zur Betreibung des erfindungsgemäßen Bestrahlungsgerätes und die Ausbildung des Strahlers und die Führung des Luftstromes, wie im vorstehenden erläutert, ausgeführt sein können.

Die Erfindung umfaßt auch alle im Sinne der Erfindung gleichwirkenden Merkmale und Ausführungsformen.

Die erfindungsgemäße Zusatzvorrichtung gemäß Fig. 15 kann inbesondere dazu verwendet werden, bestimmte kleine Objekte insbesondere Körperteile, zu bestrahlen. Durch einen flexiblen Schlauch 303 können dabei auch schwer zugängige Körperteile erreicht werden. Der die Luftströmung 302 erzeugende Fön kann dabei ortsfest angeordnet sein, weil die Strahlungsquelle am Kopfbereich des flexiblen Schlauches 303 angeordnet ist und die Luftströmung durch den Schlauch 303 geleitet wird.

Im wesentlichen besteht die Zusatzvorrichtung aus dem Kanal 301, in den durch die rückwärtige Öffnung 304 der Luftstrom 302 eines Föns (nicht dargestellt) eingeleitet wird. Die Temperatur des Luftstroms sowie Menge des Luftstroms sind vorzugsweise variabel. Im Bereich der vorderen Öffnung 305 des Kanals 301 ist mindestens eine UV-Strahlenquelle 306 vorgesehen, die

- 29 -

in Sockeln 307 gehalten wird. Elektrische Leitungen 308 verlaufen von den Sockeln 307 zur rückwärtigen Öffnung 304; von hier aus erfolgt die Stromversorgung über Kabel mit Stecker oder dgl.

In Strömungsrichtung der Luft vor der Strahlenquelle 306 sind Reflektoren 309 angebracht, die vorzugsweise mit Löchern 310 versehen sind; durch die Löcher 310 kann die Luft strömen, die Strahlenquelle 306 umspülen und mit der Strahlung auf das Objekt geleitet werden.

Gemäß Fig. 16 sind in dem Kanal 301 zwei Strahler angeordnet, die durch jeweils eine Strahlenquelle 306 und einen Reflektor 309 gebildet werden. Seitlich neben einem dieser Strahler ist ein Behältnis 311 angeordnet. Die Wandung des Behältnisses 311 ist gas- und/oder dampfdurchlässig, so daß der Luftstrom 302 hindurchströmen und dabei einen oder mehrere gasförmige oder dampfförmige Zusatzstoffe aufnehmen kann aus einem Produkt, das sich im Behältnis 311 befindet. Zu diesem Zweck kann das Behältnis 311 vorzugsweise ein Innenteil aufweisen, das als auswechselbarer Siebeinsatz ausgebildet ist, so daß man mit dem Siebbehälter einen das zu befördernde Gas bzw. den zu befördernden Dampf speichernden bzw. einen ein Gas oder Dampf abgebenden Stoff in das Behältnis 311 einsetzen kann. Die Pfeile in Fig. 16 zeigen die Strömungsrichtung des Luftstroms 302. Entsprechend den durchgezogen gekennzeichneten Pfeilen gelangt der Luftstrom 302 zu den Strahlern 306, 309 und zum Behältnis 311 (s. Pfeil 302a). Der Luftstrom 302 dringt in das Behältnis 311 ein, lädt sich mit dem zu transportierenden Zustzstoff, z.B. einem Duftstoff oder Öl oder dgl. auf und verläßt das Behältnis in Richtung auf das zu behandelnde Objekt. Da der benachbarte Luftstrom 302 auch gleichzeitig die Strahlenquelle 306 umspült und nachfolgend in Richtung der UV-Strahlen auf das zu bestrahlende Objekt strömt, ergibt sich eine kombinierte Wirkung aus einer UV-Strahlung mit einer integrierten Luftströmung und einer integrierten Luft-Gas-, bzw. Luft-Dampfstoff-Strömung. Zusätzlich zu der UV-Strahlung gelangen also auch noch die sich in dem Behältnis 311 gespeicherten Zusatzstoffe gas- oder dampfförmig auf das Objekt. Dabei können bekannte Mittel vorgesehen sein, um die Durchlässigkeit des Behältnisses zu verändern.

Um die Vielfalt der Anwendung des erfindungsgemäßen Geräts noch zu erweitern, ist zweckmäßigerweise vorgesehen, daß der Luftstrom 302 im Kanal 301 umgelenkt werden kann. Zu diesem Zweck ist eine Wandung 312 im Kanal 301 schwenkbar, und zwar in Strömungsrichtung des Luftstroms 302 vor den Strahlern 306 und dem Behältnis 311 angeordnet. Die Wandung 312 ist vorzugsweise der Querschnittskontur des Kanals 301 angepaßt, so daß sie bestimmte Wege des Luftstroms 302 versperren kann. Sie sitzt daher zweckmäßigerweise senkrecht zur Strömungsrichtung des Luftstroms 302 im Kanal 301 und ist um die Achse 316 schwenkbar gelagert. Vorzugsweise befindet sich die Schwenkachse 316 an der vorderen Kante der Wandung 312 im seitlichen Abstand von einer Wandung des Gehäuses 301, und zwar außermittig (s. Fig. 16). Mit einem außen auf der Wandung des Gehäuses 301 sitzenden, mit der Achse 316 verbundenen Verstellmittel, z.B. einem Drehknopf, kann die Wandung 312
um die Achse 316 verschwenkt werden. Die Wandung 312
ist vorzugsweise so lang ausgeführt, daß sie sowohl
gegen die linke als auch gegen die rechte Wandung ausschlagend verschwenkt werden kann (Fig. 16). Will
man z.B. verhindern, daß der Luftstrom 302 zum Behältnis 311 und den Strahlern 306, 309 gelangt, wird die
Wandung 312 aus der mit durchgezogenen Linien gezeichneten Position in die mit gestrichelten Linien gezeichnete Position verschwenkt. Das Gerät wirkt dann als
UV-Strahler mit einem seitlich aus dem Gerät austretenden Luftstrom, der zweckmäßigerweise mit an sich
bekannten Mitteln direkt auf das zu behandelnde Objekt
gelenkt wird. Da der Luftstrom 302 in diesem Fall nicht
zum Behältnis 311 gelangen kann, ist die Funktion des
Behältnisses 311 ausgeschaltet.

Die Funktion des erfindungsgemäßen Geräts wird zweckmäßigerweise durch eine weitere im Kanal 301 schwenkbar gelagerte Wandung 312a erweitert. Diese Wandung
312a ist vorzugsweise ebenfalls der Querschnittskontur
des Kanals 301 angepaßt, um die zur Achse 316 parallel
verlaufende Achse 314 schwenkbar angeordnet und befindet sich im Seitenwandungsbereich des Kanals 301 vor
dem Behältnis 311. Sie ist wesentlich kürzer ausgeführt
als die Wandung 312 und lediglich so lang, daß sie
in der gestrichelt gezeichneten Position das Behältnis
311 gegenüber dem Luftstrom 302 derart abdeckt, daß
das Behältnis 311 vor dem Luftstrom geschützt ist.
Zu diesem Zweck kann vorzugsweise noch eine in Strömungsrichtung ausgerichtete feststehende Wandung 324 zwischen dem Behältnis 311 und dem benachbarten Strahler 306, 309 angeordnet sein, gegen die die Wandung 312a verschwenkt werden kann, so daß daraus eine noch bessere Abdichtung des Behältnisses 311 gegenüber dem Luftstrom 302 resultiert. Die Wandung 312a ist zweckmäßigerweise ebenso wie die Wandung 312 gelagert, wobei die Drehachse mit einem außen auf dem Gehäuse 301 angeordneten Betätigungsmittel verbunden ist. Will man also verhindern, daß der Luftstrom 302 das Behältnis 311 erreicht, wird die Wandung 312a derart vor das Behältnis 311 verschwenkt, daß der Luftstrom 302 von der Wandung 312a in Richtung auf die Strahler 306, 309 abgelenkt wird.

Nach einer weiteren Ausführungsform der Erfindung erstreckt sich von der Achse 316 ausgehend, eine feststehende Trennwand 318 zur Öffnung 304 des Kanals 301, so daß ein Teilkanal 322 gebildet wird. Dieser Teilkanal 322 verhindert im Zusammenwirken mit der Wandung 312, daß der Luftstrom 302 zu den Strahlern 306, 309 gelangt, wenn die Wandung 312 in die gestrichelt gezeichnete Position verschwenkt wird. Zweckmäßig kann sein, die Form der Trennwand 318 so zu gestalten, daß der Luftstrom 302 vollständig vom zu bestrahlenden Objekt abgelenkt wird.

Im Rahmen der Erfindung wird ein Verfahren verwirklicht, bei dem die UV-Strahlung insbesondere deren Intensität verändert wird, indem die Strahlenquelle im Bereich eines Gases angeordnet ist und vom Gas umspült werden kann. Dabei wird an der Strahlenquelle

- 33 -

eine Gasströmung vorbeigeführt, die eine Art Gaspolster oder Gaswolke vor der Strahlenquelle bildet. Die Gasströmung kann als gerichteter Gasstrahl in die Richtung der UV-Strahlung gelenkt werden, wobei für die Gasströmung ein Spülgas verwendet werden kann. Das Spülgas ist insbesondere Luft. Vorteilhaft ist, wenn die Gasspülungen und/oder die Gasströmungen periodisch mit der UV-Strahlung kombiniert werden, und wenn vor der Strahlenquelle mit dem Spülgas ein Gasstau gegenüber der Außenatmosphäre erhöhten statischen Drucks erzeugt wird. Es kann zweckmäßig sein, die Gasart zu wechseln oder für das Spülgas und den Gasstrom unterschiedliche Gase zu verwenden. Die Menge des Gases sowie der Staudruck und der dynamische Druck des Gasstrahls können zweckmäßigerweise variiert werden. Ebenso zweckmäßig ist, Gase mit unterschiedlichen Temperaturen zu verwenden. Es war überraschend, daß dadurch, daß ein fließendes Gas in den Strahlengang zwischen Strahlenquelle und zu bestrahlendem Objekt gebracht wird, und wenn außerdem das Gas in Form einer Gasströmung auf das Objekt geleitet wird, und zwar in Richtung der UV-Strahlung, eine Wirkung erzielt werden kann, die vorher unbekannt war. Es hat sich gezeigt, daß die Wirkung der Strahlung verändert wird. Beispielsweise ist die Heilung bei Hauterkrankungen wesentlich besser; es erfolgt eine schnellere Ausmerzung z.B. der Schuppenflechte, wenn Luft für die Spülung und den Gasstrahl verwendet wird; aber auch andere Hauterkrankungen werden schneller geheilt. Worauf diese veränderte Wirkung der Strahlung einer Strahlenquelle beruht, ist derzeit noch unbekannt. Möglicherweise wird die Zusammensetzung des Luftgasgemisches verändert und/ oder die Gasmenge, die pro Zeiteinheit auf das Objekt befördert wird, wirkt förderlich und/oder die Filterwirkung des verwendeten Spülgases und/oder des Gases für den Gasstrahl bedingt die überraschende Wirkung. Eine günstige Wirkung kann schon dann erzielt werden, wenn ein strömendes Gas durch den Strahlengang zwischen Strahlungsquelle und zu bestrahlendem Objekt geführt wird. Die Gasströmung kann senkrecht zur Bestrahlungsrichtung oder geneigt bis in Strahlungsrichtung gelenkt werden. Die Erfindung sieht vor, daß die Gasart, die Gasmenge, die Gasströmungsrichtung, die Gastemperatur sowie der Staudruck und der dynamische Druck des Gases variabel sind. Es kann zweckmäßig sein, das Gas schichtartig senkrecht oder geneigt durch den Raum zwischen Strahlungsquelle und zu bestrahlendem Objekt zu führen, wobei ebenfalls insbesondere die Schichtstärke, die Strömungsgeschwindigkeit, die Gasart und die Gastemperatur sich nach der gewünschten Veränderung der Strahlung richten. In Kombination mit den erfindungsgemäßen Veränderungsmöglichkeiten der Strahlung können an sich bekannte Veränderungsmöglichkeiten wie z.B. zu- oder abschaltbare Strahlungsquellen und/oder Filter eingesetzt werden. Bei großflächigen Strahlern wie Solarien kann die Erfindung ebenfalls mit Erfolg verwirklicht werden, indem zonal im Strahler Gasströme erzeugt werden, und zwar in Bereichen, in denen das zu bestrahlende Objekt mit unterschiedlichen Intensitäten bestrahlt werden soll.

0104466

M 149/+I/kn

Johann Josef Kerschgens
Prinz-Ludwig-Str. 5, 8918 Diessen am Ammersee

Patentansprüche:

1. Bestrahlungsgerät, kombiniert aus einem Gebläse
und einer Strahlungsquelle, die ultraviolette Strahlung (UV-Strahlung) abgibt und über einen Vorwiderstand an die Netzspannung eine Wechselspannungsquelle
angeschlossen ist, wobei die Strahlungsquelle mit
dem vom Gebläse erzeugten Luftstrom zusammenwirkt,
d a d u r c h   g e k e n n z e i c h n e t ,  daß
der Vorwiderstand (41,94) im wesentlichen kapazitiv
ist und daß die Strahlungsquelle (33) im Luftstrom
des Gebläses (140) angeordnet ist, wobei der Luftstrom die Strahlungsquelle derart kühlt, daß der
Momentanwert ihrer Wiederzündspannung $\leq$ dem Momentanwert der Netzspannung ist.

2. Bestrahlungsgerät nach Anspruch 1, d a d u r c h
g e k e n n z e i c h n e t ,  daß das Gebläse (140)
ein Haarfön (141) ist, auf dessen Luftaustrittsstutzen (142) ein Gehäuse (1) aufsetz- und arretierbar angeordnet ist, das die Strahlungsquelle (33)
und den Vorwiderstand (41,94) aufweist.

3. Bestrahlungsgerät nach einem oder mehreren der vorhergehenden Ansprüche, d a d u r c h   g e k e n n z e i c h n e t, daß eine am Gehäuse (1) angebrachte Aufstecktülle (22) paßgerecht mit dem Luftaustrittsstutzen (142) des Föns (141) in Eingriff bringbar ist.

4. Bestrahlungsgerät nach einem oder mehreren der vorhergehenden Ansprüche, d a d u r c h   g e - k e n n z e i c h n e t, daß in dem Gehäuse (1) Luftführungskanäle (24, 25) ausgebildet sind, die ausgehend von der Öffnung (23) der Aufsteckhülle (22) den Luftstrom entlang der Kondensatoren ( 9, 10; 95, 96, 97) des kapazitiven Vorwiderstandes (41, 94) und der UV-Strahlungsquelle (33) und/oder der Fassung (30) der UV-Strahlungsquelle (33) führen.

5. Bestrahlungsgerät nach Anspruch 4 , d a d u r c h g e k e n n z e i c h n e t, daß in das Gehäuse (1) Auslässe (26, 27, 28, 29) für die Abfuhr der Kühlluft eingelassen sind.

6. Bestrahlungsgerät nach einem oder mehreren der vorhergehenden Ansprüche, d a d u r c h   g e - k e n n z e i c h n e t, daß der Reflektor (34) mehrere ebene Flächen (35, 36, 37, 38, 39) aufweist.

7. Bestrahlungsgerät nach Anspruch 6 , d a d u r c h g e k e n n z e i c h n e t, daß die ebenen Flächen (35, 36, 37, 38, 39) jeweils unter einem Winkel

- 3 -

zueinander derart angeordnet sind, daß ihre Hauptreflexionsrichtungen etwa auf eine begrenzte
Bestrahlungsfläche gerichtet sind.

8. Bestrahlungsgerät nach Anspruch 6 und/oder 7,
d a d u r c h   g e k e n n z e i c h n e t, daß
der Reflektor (34) aus Kunststoff besteht.

9. Bestrahlungsgerät nach einem oder mehreren der
vorhergehenden Ansprüche, d a d u r c h   g e-
k e n n z e i c h n e t, daß die Oberfläche des
Reflektors (34) metallbeschichtet ist.

10. Bestrahlungsgerät nach Anspruch 9 , d a d u r c h
g e k e n n z e i c h n e t, daß die Oberflächenschicht aus Edelstahl und/oder aus Chrom besteht.

11. Bestrahlungsgerät nach Anspruch 9., d a d u r c h
g e k e n n z e i c h n e t, daß die Oberflächenschicht aus gelbem,eloxiertem Aluminium besteht.

12. Bestrahlungsgerät nach einem oder mehreren der vorhergehenden Ansprüche, d a d u r c h   g e k e n n-
z e i c h n e t, daß der Reflektor (34) eine
bevorzugt UV-A-Strahlung reflektierende Oberflächenschicht aufweist.

13. Bestrahlungsgerät nach einem oder mehreren der
vorhergehenden Ansprüche, d a d u r c h   g e-

- 4 -

k e n n z e i c h n e t, daß der Reflektor (34)
Kühlluftdurchtrittsöffnungen (223) zur.Zuführung von
Kühlluft des Gebläses zur Strahlungsquelle (33)
aufweist.

14. Bestrahlungsgerät nach einem oder mehreren der
vorhergehenden Ansprüche, d a d u r c h  g e-
k e n n z e i c h n e t, daß die Strahlungsquelle (33) eine Quecksilberdampf-Hochdrucklampe
(33) (UV-Lampe) ist.

15. Bestrahlungsgerät nach Anspruch14 , d a d u r c h
g e k e n n z e i c h n e t, daß eine einen
hohen Anteil an langwelliger UV-A-Strahlung
abgebende UV-Lampe (33) verwendet wird.

16. Bestrahlungsgerät nach einem oder mehreren der
vorhergehenden Ansprüche, d a d u r c h  g e-
k e n n z e i c h n e t, daß das Spektrum der
emittierten Strahlung der UV-Lampe (33) im wesentlichen im Wellenlängenbereich von etwa 240 nm
bis etwa 585 nm liegt.

17. Bestrahlungsgerät nach einem oder mehreren der
vorhergehenden Ansprüche, g e k e n n z e i c h-
n e t  d u r c h  eine in den Strahlengang des
Bestrahlungsgerätes anordbare, kurzwellige UV-B-
und UV-C-Strahlung unterdrückende Filtervorrichtung.

- 5 -

18. Bestrahlungsgerät nach einem oder mehreren der vorhergehenden Ansprüche, g e k e n n z e i c h - n e t d u r c h eine Filtervorrichtung, die langwellige sichtbare Strahlung und IR-Strahlung unterdrückt.

19. Bestrahlungsgerät nach einem oder mehreren der vorhergehenden Ansprüche, d a d u r c h g e - k e n n z e i c h n e t, daß der kapazitive Vorwiderstand (41, 94) wenigstens einen Kondensator (9, 10; 95, 96, 97) aufweist, der in Reihe zur UV-Lampe (33) geschaltet ist.

20. Bestrahlungsgerät nach Anspruch 19, d a d u r c h g e k e n n z e i c h n e t, daß der kapazitive Vorwiderstand (41, 94) mehrere Kondensatoren (9; 10; 95, 96, 97) aufweist, die zueinander parallel geschaltet sind.

21. Bestrahlungsgerät nach einem oder mehreren der vorhergehenden Ansprüche, g e k e n n z e i c h - n e t d u r c h einen parallel zum Kondensator bzw. zu den Kondensatoren (9, 10; 95, 96, 97) geschalteten Entladewiderstand (45, 99).

22. Bestrahlungsgerät nach einem oder mehreren der vorhergehenden Ansprüche, d a d u r c h g e - k e n n z e i c h n e t, daß der Vorwiderstand (41, 94) einen in Reihe zum Kondensator bzw.

- 6 -

zu den Kondensatoren (9, 10; 95, 96, 97) geschalteten Widerstand (42, 98) zur Spitzenstrombegrenzung aufweist.

23. Bestrahlungsgerät nach Anspruch 22, d a d u r c h g e k e n n z e i c h n e t, daß der Entladewiderstand (45, 99) parallel zu der Reihenschaltung aus Spitzenstrombegrenzungswiderstand (42, 98) und Kondensator bzw. Kondensatoren (9, 10; 95, 96, 97) geschaltet ist.

24. Bestrahlungsgerät nach einem oder mehreren der vorhergehenden Ansprüche, d a d u r c h g e k e n n z e i c h n e t, daß der Fön (141) einen Motor (83) und eine Heizwicklung (79) aufweist, wobei in einer ersten Betriebsart des Fönes (141) die Heizwicklung (79) an Betriebsspannung liegt und der Motor (83) an einem Teilabschnitt der Heizwicklung (79) angeschlossen ist.

25. Bestrahlungsgerät nach Anspruch 24, d a d u r c h g e k e n n z e i c h n e t, daß der eine Motoranschluß (124) an einem Abgriff (81) der Heizwicklung (79) und der andere Motoranschluß (125) an einem Ende (126) der Heizwicklung (79) angeschlossen ist.

26. Bestrahlungsgerät nach Anspruch 24 und/oder 25, d a d u r c h g e k e n n z e i c h n e t, daß der Fön (141) eine zweite Betriebsart aufweist, bei der wenigstens ein Teil der Heizwicklung
(79) als Vorwiderstand für den Motor (83) eingesetzt wird.

27. Bestrahlungsgerät nach Anspruch 26, d a d u r c h
g e k e n n z e i c h n e t, daß ein Motoranschluß (124) an dem Abgriff (81) der als Vorwiderstand dienenden Heizwicklung (79) angeschlossen
ist und die Versorgungsspannung für den Fön (141)
zwischen dem anderen Motoranschluß (125) und
dem Anfang (127) der Heizwicklung (79) liegt.

28. Bestrahlungsgerät nach einem oder mehreren der
vorhergehenden Ansprüche, d a d u r c h g e-
k e n n z e i c h n e t, daß der Motor ein Gleichstrommotor (83) ist und über eine Diode (82)
mit dem Abgriff (81) der Heizwicklung (79) in
Verbindung steht.

29. Bestrahlungsgerät nach einem oder mehreren der
vorhergehenden Ansprüche, d a d u r c h g e-
k e n n z e i c h n e t, daß in Reihe zu der
als Vorwiderstand dienenden Heizwicklung (79)
ein Thyristor (109) einer Phasenanschnittsschaltung
(73) zum Regeln der Motordrehzahl des Motors
(83) geschaltet ist.

30. Bestrahlungsgerät nach Anspruch 29, d a d u r c h
g e k e n n z e i c h n e t, daß das phasenanschnittswinkelbestimmende Glied (107) der Phasen-

- 8 -

anschnittsschaltung (73) mindestens einen NTC-Widerstand (112, 113) aufweist, der zur Temperaturermittlung der UV-Lampe (33) in'deren Nähe angeordnet ist.

31. Bestrahlungsgerät nach Anspruch 30, d a d u r c h g e k e n n z e i c h n e t, daß das phasenanschnittswinkelbestimmende Glied (107) mehrere NTC-Widerstände aufweist, die jeweils einer UV-Lampe zugeordnet sind.

32. Bestrahlungsgerät nach Anspruch 30 und/oder 31, d a d u r c h g e k e n n z e i c h n e t, daß zu dem NTC-Widerstand bzw. zu den NTC-Widerständen (112, 113) ein Motordrehzahleinstelltrimmer (114) zur Arbeitspunkteinstellung (Temperatureinstellung) der UV-Lampe(n) in Reihe geschaltet ist.

33. Bestrahlungsgerät nach einem oder mehreren der vorhergehenden Ansprüche, d a d u r c h g e k e n n z e i c h n e t, daß die Motordrehzahl des Föns durch die Phasenanschnittsschaltung (73) derart geregelt wird, daß die UV-Lampe (33) bzw. UV-Lampen eine konstante, vorwählbare Temperatur aufweist bzw. aufweisen.

34. Bestrahlungsgerät nach einem oder mehreren der vorhergehenden Ansprüche, g e k e n n z e i c h n e t d u r c h eine die UV-Lampe (33) bzw.

0104466

- 9 -

die UV-Lampen an- bzw. ausschaltende Zeitschaltung (71) für die genaue Dosierung der Bestrahlung.

35. Bestrahlungsgerät nach Anspruch 1 oder einem oder mehreren der Ansprüche 3 bis 34, d a d u r c h g e k e n n z e i c h n e t, daß das Gebläse (140) ein Haarfön (141) ist, in dessen Luftaustrittsstutzen (142) die Strahlungsquelle angeordnet ist und der Vorwiderstand (41; 91) und die übrigen Schaltungselementen innerhalb des Föngehäuses verteilt angeordnet sind.

36. Bestrahlungsgerät nach einem oder mehreren der vorhergehenden Ansprüche, d a d u r c h g e - k e n n z e i c h n e t, daß auf den Luftaustrittsstutzen des Haarföns ein flexibler Schlauch (216) mit einer Tülle (217) aufsetzbar ist und am der Tülle gegenüberliegenden Ende des flexiblen Schlauches ein mit Luftaustrittsschlitzen (220) versehener Ansatz befestigt ist, der zum Anschluß des die Strahlungsquelle aufweisenden Aufsatzes (143) dient.

37. Bestrahlungsgerät nach Anspruch 36, d a d u r c h g e k e n n z e i c h n e t, daß in dem flexiblen Schlauch (216) eine elektrische Verbindungsleitung integriert ist, die zur Verbindung des Föns (141) mit dem Aufsatz (143) dient.

38. Bestrahlungsgerät nach einem oder mehreren der vorhergehenden Ansprüche, d a d u r c h g e - k e n n z e i c h n e t, daß der Luftaustrittsbereich des UV-Strahlers durch Luftaustrittsöffnungen (223) gebildet wird, die in Strömungsrichtung gesehen vor der UV-Lampe (33) angeordnet sind und über die gesamte Länge der Lampe verteilt sind und beidseitig des Strahlers parallel zu der UV-Lampe, einander gegenüberliegend, Luftaustrittsschächte (224) ausgebildet sind.

39. Bestrahlungsgerät nach einem oder mehreren der vorhergehenden Ansprüche, d a d u r c h  g e - k e n n z e i c h n e t, daß an das Föngehäuse (205) ein bügelförmiger Handgriff (206) integriert geformt ist, der eine mittlere Durchgriffsöffnung (206a) aufweist, so daß sich eine günstige Gewichtsverteilung und Aufteilungsmöglichkeit für die einzelnen Bauelemente in den verbleibenden Griffschenkeln ergibt.

40. Bestrahlungsgerät nach einem oder mehreren der vorhergehenden Ansprüche, d a d u r c h  g e - k e n n z e i c h n e t, daß unterhalb des Luftaustrittsstutzens im Gehäuse ein Anschlußstecker (202) für den elektrischen Anschluß des Strahlungsaufsatzes ausgebildet ist.

41. Bestrahlungsgerät nach einem oder mehreren der vorhergehenden Ansprüche, g e k e n n z e i c h - n e t  d u r c h  einen Gaskanal (301) mit einer hinteren Öffnung (304) und einer Mündung (305), wobei im Bereich der Mündung (305) mindestens eine Strahlenquelle (306) vorzugsweise auswechselbar derart angeordnet ist, daß deren Strahlung aus der Mündung nach vorne weist, die hintere Öffnung (304) des Gaskanals (301) mit einer Einrichtung verbunden ist, die eine Gasströmung erzeugt und dafür gegebenenfalls an eine Gasquelle angeschlossen ist, wobei die Gasströmung in den Gaskanal (301) gedrückt wird, und wobei die Strahlenquelle (306) so positioniert ist, daß sie vom Gas umspült werden und der Gasstrom danach als gerichteter Gasstrahl aus der Mündung (305) austreten kann.

42. Bestrahlungsgerät nach Anspruch 41, d a d u r c h g e k e n n z e i c h n e t, daß die Strahlenquelle (306) in Sockeln (307) steckt, die elektrische Elemente aufweisen, die die Strahlenquelle (306) mit elektrischer Energie versorgen, wobei elektrische Leitungen (308) von den Sockeln (307) zur rückwärtigen Öffnung (304) oder an die Außenseite des Gaskanals (301) vorzugsweise zu Steckerelementen führen, wo sie mit erforderlichen anderen elektrischen Stecker- und Schaltelementen verbindbar angeordnet sind.

43. Bestrahlungsgerät nach Anspruch 41 und/oder 42, d a d u r c h g e k e n n z e i c h n e t, daß hinter der Strahlenquelle (306) ein Reflektor (309) angeordnet ist, der Löcher (310) oder dgl. Öffnungen aufweist.

44. Bestrahlungsgerät nach einem oder mehreren der Ansprüche 41 bis 43, d a d u r c h g e k e n n - z e i c h n e t, daß die Löcher (310) in der Größe veränderbar, vorzugsweise verschließbar, angeordnet sind.

45. Bestrahlungsgerät nach einem oder mehreren der Ansprüche 41 bis 43, d a d u r c h g e k e n n - z e i c h n e t, daß vor der Strahlenquelle (306) in der Mündung (305) des Gaskanals (301) eine Stauplatte (311) aus UV-Strahlung durchlässigem Material angeordnet ist.

46. Bestrahlungsgerät nach Anspruch 45, d a d u r c h
g e k e n n z e i c h n e t, daß die Stauplatte
(311) aus Filtermaterial für bestimmte Wellenbereiche der UV-Strahlung besteht.

47. Bestrahlungsgerät nach Anspruch 45 und/oder 46,
d a d u r c h   g e k e n n z e i c h n e t, daß
zwischen der Stauplatte (311) und dem Reflektor (309)
mindestens ein Spalt (312) vorgesehen ist.

48. Bestrahlungsgerät nach einem oder mehreren der Ansprüche 45 bis 47, d a d u r c h   g e k e n n -
z e i c h n e t, daß die Stauplatte (311) auswechselbar im Bereich der Mündung (305) im Gaskanal (301)
angeordnet ist.

49. Bestrahlungsgerät nach einem oder mehreren der Ansprüche 41 bis 48, g e k e n n z e i c h n e t
d u r c h   an sich bekannte Mittel zur Variierung
des Gasdrucks des einströmenden Gases (302) sowie
dessen Temperatur.

50. Bestrahlungsgerät nach einem oder mehreren der Ansprüche 41 bis 49, d a d u r c h   g e k e n n -
z e i c h n e t, daß zum Schutz der Sockel (307)
in der Mündungsöffnung (305) vor den Sockeln (307)
jeweils eine Metallblende (313) angeordnet ist.

51. Bestrahlungsgerät nach einem oder mehreren der Ansprüche 41 bis 50, d a d u r c h   g e k e n n -
z e i c h n e t, daß die hintere Öffnung (304)
und die Gas- bzw. Gasströmungsquelle über einen
flexiblen Schlauch (303) miteinander verbunden sind.

52. Bestrahlungsgerät nach einem oder mehreren der Ansprüche 41 bis 51, g e k e n n z e i c h n e t d u r c h einen Strahler (315) eines großflächigen Solariums (314) mit mehreren Strahlenquellen (306), die vorzugsweise mit Reflektoren (309) umgeben sind, wobei Gaszuleitungen (316) vorgesehen sind, die mit Gaskanälen (301) in Verbindung stehen, wobei die Gaskanäle (301) die Reflektoren und Strahlenquellen (306) umgeben, so daß ein Gasstrom (317) gebildet werden kann, der im wesentlichen parallel zur Strahlung (318) der jeweiligen Strahlenquelle (306) verläuft.

53. Bestrahlungsgerät nach Anspruch 52, d a d u r c h g e k e n n z e i c h n e t, daß die Reflektoren (309) Durchströmöffnungen (310) aufweisen.

54. Bestrahlungsgerät nach Anspruch 52 und/oder 53, d a d u r c h g e k e n n z e i c h n e t, daß alle Strahlenquellen (306) mit Gaskanälen (301) ausgerüstet sind, von denen einzelne oder alle in bezug auf die Gaszuführung absperrbar angeordnet sind.

55. Bestrahlungsgerät nach einem oder mehreren der vorhergehenden Ansprüche, g e k e n n z e i c h n e t d u r c h mindestens eine im Kanal (301) vor einem Strahler (306, 309) verschwenkbar angeordnete, den Luftstrom (302) zumindest teilweise ablenkende Wandung (312).

56. Bestrahlungsgerät nach Anspruch 55, g e k e n n z e i c h n e t d u r c h ein seitlich neben einem Strahler (306, 309) angeordnetes Behältnis (311) mit einer gas- und/oder dampfdurchlässigen Wandung.

57. Bestrahlungsgerät nach Anspruch 55 und/oder 56, d a d u r c h   g e k e n n z e i c h n e t, daß es einen Kanal (301) aufweist, in dem durch die rückwärtige Öffnung (304) ein Luftstrom (302) eines Föns einleitbar ist, im Bereich der vorderen Öffnung (305) des Kanals (301) mindestens eine UV-Strahlenquelle (306) sitzt, die in Sockeln (307) gehalten wird, wobei elektrische Leitungen (308) vor den Sockeln (307) zur rückwärtigen Öffnung (304) laufen, und daß in Strömungsrichtung der Luft vor der Strahlenquelle (306) Reflektoren (309) angebracht sind, die vorzugsweise mit Löchern versehen sind, so daß durch die Löcher (310) Luft strömen und die Strahlenquelle (306) umspülen kann.

58. Bestrahlungsgerät nach einem oder mehreren der Ansprüche 55 bis 57, d a d u r c h   g e k e n n z e i c h n e t, daß seitlich neben dem Strahler (306) ein Behältnis (311) angeordnet ist, dessen Wandung gas- und/oder dampfdurchlässig ist, wobei das Behältnis (311) vom Luftstrom (302) erfaßt werden kann.

59. Bestrahlungsgerät nach Anspruch 58, d a d u r c h   g e k e n n z e i c h n e t, daß sich im Behältnis (311) ein Innenteil befindet, das als auswechselbarer Siebeinsatz ausgebildet ist.

60. Bestrahlungsgerät nach einem oder mehreren der Ansprüche 55 bis 59, d a d u r c h   g e k e n n z e i c h n e t, daß eine Wandung (312) im Kanal (301) schwenkbar in Strömungsrichtung des Luftstroms (302) vor den Strahlern (306) und dem Behältnis (311) angeordnet ist, wobei die Wandung (312) der Querschnittskontur des Kanals (301) angepaßt ist.

61. Bestrahlungsgerät nach Anspruch 60, d a d u r c h
g e k e n n z e i c h n e t, daß die Wandung (312)
senkrecht zur Strömungsrichtung des Luftstroms (302)
im Kanal (301) um die Achse (316) schwenkbar gelagert ist.

62. Bestrahlungsgerät nach Anspruch 61, d a d u r c h
g e k e n n z e i c h n e t, daß sich die Schwenkachse (316) an der vorderen Kante der Wandung (312)
im seitlichen Abstand von einer Wandung des Gehäuses (301) außermittig befindet.

63. Bestrahlungsgerät nach einem oder mehreren der Ansprüche 60 bis 62, d a d u r c h   g e k e n n -
z e i c h n e t, daß die Wandung (312) so lang
ausgeführt ist, daß sie sowohl gegen die linke als
auch gegen die rechte Wandung des Gehäuses (301)
verschwenkt werden kann.

64. Bestrahlungsgerät nach einem oder mehreren der Ansprüche 55 bis 63, g e k e n n z e i c h n e t
d u r c h   eine weitere, im Kanal (301) schwenkbar
gelagerte Wandung (312a), die ebenfalls der Querschnittskontur des Kanals (301) angepaßt und um die
Achse (314) schwenkbar angeordnet ist und sich im
Seitenwandungsbereich des Kanals (301) vor dem Behältnis (311) befindet.

65. Bestrahlungsgerät nach Anspruch 64, d a d u r c h
g e k e n n z e i c h n e t, daß eine feststehende
Wandung (324) zwischen dem Behältnis (311) und dem
benachbarten Strahler (306, 309) angeordnet ist.

66. Bestrahlungsgerät nach einem oder mehreren der Ansprüche 55 bis 65, d a d u r c h   g e k e n n -
z e i c h n e t, daß sich von der Achse (316) ausgehend, eine feststehende Trennwand (318) zur Öffnung (304) des Kanals (301) erstreckt.

67. Bestrahlungsgerät nach einem oder mehreren der vorangehenden Ansprüche, g e k e n n - z e i c h n e t d u r c h einen am Gehäuse (1, 301) des UV-Aufsatzes (143) befestigten Abstandshalter (128).

68. Bestrahlungsgerät nach Anspruch 67, d a - d u r c h g e k e n n z e i c h n e t , daß der Abstandhalter (128) aus einem Gitter (129) aus Kunststoff oder Draht besteht, das etwa die Größe der dem Bestrahlungsobjekt zugewandten Seite des UV-Aufsatzes (143) besitzt, wobei am Gitter (129) einendig Abstandsstreben (130) befestigt sind, die rechtwinklig zu der Gitterebene verlaufen und anderendig in Richtung der Längsachse (11) verschieblich sowie arretierbar an dem Gehäuse (1) geführt sind.

69. Bestrahlungsgerät nach Anspruch 68, d a - d u r c h g e k e n n z e i c h n e t , daß die Randbereiche (131) des Gitters (129) in Richtung auf das Gehäuse (1) abgewinkelt sind.

70. Bestrahlungsgerät nach Anspruch 69, d a - d u r c h g e k e n n z e i c h n e t , daß an einer Abstandsstrebe (130) Stricheinteilungen (132) angeordnet sind.

71. Bestrahlungsgerät nach Anspruch 70, g e - k e n n z e i c h n e t d u r c h ein

- 17 -

an dem Gehäuse (1) drehbar gelagertes Zahnrad (133), das mit einer Zahnung (134) einer der Abstandsstreben (130) kämmt, wobei das Zahnrad mit einem Bedienknopf (135) verbunden ist, der außerhalb des Gehäuses (1) angeordnet ist.

FIG.1

M 149

FIG.2

34

37

39    33    39

38

36

-2/12-

0104466

M 149

FIG.3

# FIG.4

220V/ 50 Hz

# FIG.5

FIG.5

58

1N 4001

LM317

270 Ω

1N 4001

59 3-20V
2A

51 1A

53

100n

54
4x
1N 4001

55

56

100 Ω

57

61

5kΩ

60
0V

7809

+9V 1A

7806

+6V 1A

52

M 149

-5/12-

0104466

FIG.6

24 x LED  GL 9  P 4  od CQV

$U_{DD}$ 15V
(8–18V)

62

63

65
RT

66
ZD
5,6 V

1k

2x
100k

100k

18 17 16 15 14 13 12 11 10
UAA 180
1 2 3 4 5 6 7 8 9

64

$U_{Motor}$

67

68

10k

2x
100k

ZP
5,6 V

18 17 16 15 14 13 12 11 10
UAA 180
1 2 3 4 5 6 7 8 9

M 149

0104466

-6/11-

FIG.7

FIG.8

FIG.9

FIG.10

FIG.12

M149

FIG.11

## FIG.13

## FIG.14

$\dfrac{12}{12}$

FIG.15

FIG. 16

Europäisches
Patentamt

**EUROPÄISCHER RECHERCHENBERICHT**

**0104466**

Nummer der Anmeldung

EP 83 10 8494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-A-1 464 661 (BAUMANN)<br><br>*Seite 2, Zeilen 12-27*<br><br>--- | 1,5-7, 12,13, 24,38, 41,43, 52,53 | A 61 N 5/06 |
| A | US-A-1 579 513 (CAMERON)<br><br>*Seite 1, Zeile 71 bis Seite 2, Zeile 110*<br><br>--- | 1,20, 24,44, 50,54 | |
| A | DE-A-2 832 143 (HIRSCH)<br><br>*Figur 1*<br><br>--- | 1,19-23 | |
| A | DE-A-2 823 615 (WEISSMANN)<br><br>*Seite 6, Zeile 23 bis Seite 13, Zeile 2*<br><br>--- | 2,29, 34,38, 42,49, 57 | RECHERCHIERTE SACHGEBIETE (Int. Cl. ³)<br><br>A 61 N 5/00 |
| A | DE-A-2 906 512 (HUBNER)<br>*Seite 3, Zeile 16; Seite 5, Zeilen 29-32*<br><br>--- | 14-17 | |
| A | DE-A-2 841 112 (NATH)<br>*Seite 8, Zeile 4*<br><br>---     -/- | 18 | |

Der vorliegende Recherchenbericht wurde für alle Patentansprüche erstellt.

| Recherchenort<br>DEN HAAG | Abschlußdatum der Recherche<br>29-11-1983 | Prüfer<br>BERTIN M.H.J. |
|---|---|---|

**0104466**

Nummer der Anmeldung

EP 83 10 8494

Europäisches
Patentamt

# EUROPÄISCHER RECHERCHENBERICHT

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-A-2 714 724 (MUTZHAS) *Figuren* ----- | 45-46, 48 | |

RECHERCHIERTE SACHGEBIETE (Int. Cl. ³)

Der vorliegende Recherchenbericht wurde für alle Patentansprüche erstellt.

| Recherchenort | Abschlußdatum der Recherche | Prüfer |
|---|---|---|
| DEN HAAG | 29-11-1983 | BERTIN M.H.J. |